# EUROPEAN PATENT APPLICATION

(11) **EP 4 275 700 A2**
(43) Date of publication of application: **15.11.2023**
(21) Application number: 23184700.5
(22) Date of filing: 11.05.2020
(51) Int. Cl.: A61K 39/015

(54) **VACCINE IMMUNOGENS**

(30) Priority: 10.05.2019 GB 201906641
(62) Divisional of application: 20728129.6
(71) Applicant: Fundação Oswaldo Cruz - FIOCRUZ, 21045-900 Rio de Janeiro RJ (BR); Oxford University Innovation Ltd., Oxford, Oxfordshire OX2 0JB (GB)
(72) Inventor: Hill, Adrian Vivian Sinton, Oxford (GB); Bettencourt, Paulo Jorge Goncalves de, Oxford (GB); Spencer, Alexandra Jane, Oxford (GB); Ternette, Nicola Maria Nathalie, Oxford (GB); Salman, Ahmed Mahmoud Ahmed Ahmed, Oxford (GB); Junqueira, Caroline Furtado, Belo Horizonte (BR); Gazzinelli, Ricardo Tostes, Belo Horizonte (BR); Barbosa, Camila Raquel Rodrigues, Belo Horizonte (BR)
(74) Representative: Weidner Stern Jeschke

(57) **Abstract**

An immunogenic composition comprising: a) one or more plasmodium-derived ribosomal or ribosomal associated protein or immunogenic fragment thereof which has a sequence which is at least about 80%, 85%, 90%, 95%, 98%, 99% or 100% identical to a ribosomal or ribosomal associated protein or an immunogenic fragment of a ribosomal or ribosomal associated protein recited in Figure 1; or a ribosomal or ribosomal associated protein or peptide or immunogenic fragment thereof as recited in Figure 2 or Figure 3; and/or b) a polynucleotide encoding one or more protein, peptide or immunogenic fragment of a); wherein the immunogenic composition is for use in eliciting an immune response in a subject to treat or prevent malaria. Also provided are
plasmodium-derived ETRAMPs and/or histones, or immunogenic fragments thereof, for use in eliciting an immune response in a subject, preferably to treat or prevent malaria.

## Description

The project leading to this application has received funding from the European Union's Horizon 2020 research and innovation programme under grant agreement No 733273.

The invention relates to immunogenic proteins or fragments thereof derived from *Plasmodium falciparum* and/or *Plasmodium vivax* for use in the treatment or prevention of malaria.

Malaria is a serious and life-threatening mosquito-borne infectious disease caused by parasitic protozoans of the genus *Plasmodium.* In 2018, there were approximately 500,000 deaths from malaria mainly caused by *Plasmodium falciparum.* Whilst preventative small molecule based medicines exist to prevent malaria, such as chloroquine, they can be associated with significant side-effects, they are unsuitable for long-term use, and drug resistance is increasingly problematic. Vaccination programs have proven to be effective in the reduction and eradication of various diseases worldwide. The aim is to develop an effective malaria vaccine, which is urgently needed. However, current single-component vaccines lack sufficient efficacy for deployment in the field. The two leading malaria vaccine candidates, RTS,S and ChAd63-MVA ME-TRAP, are both sub-unit vaccines targeting the pre-erythrocytic phase of malaria. Whilst neither vaccine currently provides optimal protective efficacy for deployment in endemic countries, they both demonstrate the strength of targeting the pre-erythrocytic phase, as no blood-stage vaccine has progressed as far in clinical development.

The most advanced vaccine in clinical development, RTS,S/AS01, has completed a phase III trial in African infants and children, where efficacy in young infants was <20% and in 5 - 17 month olds was 35% . This vaccine is based on a platform in which RTS,S has four molecules of unfused HBsAg (Hepatitis B surface antigen) for each fusion protein containing HBsAg fused to the C-terminal half of the circumsporozoite protein (CSP). The vaccine works by inducing production of a high titre of antibodies against the central four amino acid conserved repeat of the circumsporozoite protein (CSP) of the malaria sporozoites. But as antibody titres fall overtime, protection is reduced and then lost. There have also been suggestions of possible safety issues in the phase III trial and also uncertainty about logistic deployability which has led to a delay in WHO pre-qualification, licensure and deployment of this vaccine.

Vaccination with irradiated sporozoites delivered by mosquito bite has been considered the 'gold-standard' of malaria vaccines, as whilst it is impractical for deployment, this regimen has repeatedly shown sterile protection in vaccinated volunteers. The increased efficacy of irradiated sporozoite immunization over sub-unit vaccines is likely because immune responses are induced to a broad range of antigenic targets. However, perhaps not only multiple targets are needed to create an efficacious sub-unit vaccine, but also better targets than those traditionally focused on (e.g. CSP and TRAP). Over 5000 different proteins are expressed throughout the *Plasmodium* life-cycle, leading to a high probability that alternative target antigens other than CSP or TRAP may exist, or a target antigen to be used alongside CSP or TRAP in a multicomponent vaccination strategy.

Several approaches are being utilised to try and produce an improved or alternative vaccine. One such approach may be to target the pre-erythrocytic, liver stage of the life cycle of the parasite. This type of malaria vaccine, unlike those described above, targets not the sporozoites themselves but the liver-stage parasite that grows in hepatocytes for approximately seven days after sporozoites successfully infect the liver.

The second part of the pre-erythrocytic stage (the liver-stage) ends with the emergence of large numbers of merozoites from each hepatocyte seven days after infection of the hepatocyte. Typically about 20,000 parasites emerge from each hepatocyte and rapidly invade erythrocytes, to start the blood-stage of infection. If the blood-stage is not controlled rapidly, the infected individual develops malaria and may die from the disease. Clearly, pre-erythrocytic vaccines have the potential advantage of stopping the malaria infection before any blood-stage infection occurs and when the number of potentially infected host cells is small.

A particularly promising approach to malaria liver-stage vaccine development has been to use recombinant non-replication-competent viral vectors such as adenoviral vectors or attenuated vaccine vectors, for example simian or human adenoviruses and modified vaccinia Ankara (MVA). Plasma DNA vectors and RNA-based vectors have also been tested but have yet to show clear clinical efficacy when used alone, but may usefully be used in heterologous prime boost regimens with viral vectors. Viral vectors appear to be particularly effective against the liver-stage because of their ability in inducing a cellular immune response, specifically malaria-specific CD8+ T cells that can target and kill malaria-infected hepatocytes.

CD8+ T cells recognise peptide antigens that are displayed on the surface of major histocompatibility complex (MHC) class I molecules, also known as HLA class I molecules in humans, on the surface of infected cells. CD8+ T cells, which are highly cytolytic, are strongly implicated in anti-malaria immunity at the liver stage. In some vaccine trials, the number of vaccine-induced malaria-specific CD8+ T cells has been shown to correlate with liver stage vaccine efficacy in humans, consistent with many experimental animal vaccine studies which have demonstrated this protective mechanism.

Because of this correlation, a so-called heterologous prime-*boost* vaccination regime is frequently used. This involves sequential immunisation with two different viral vectors encoding the same malaria antigen(s) or epitope(s). The vectors may be replication competent but preferentially are non-replication competent. This generates higher numbers of circulating CD8+ T cells compared to use of a single vector alone or with repeated administrations and leads to much higher vaccine efficacy in many clinical trials. A recent further development of liver-stage immunisation approaches has been to use a so-called prime-*target* immunisation regime. This involves administering the last dose of viral vector by an intravenous route (or other route that leads to antigen deposition in the liver) and has been found to increase the number of malaria-specific CD8+ T cells amongst the resident memory T cell population in the liver. Importantly, this generally leads to substantially greater vaccine efficacy (see WO2017/178809).

However, a challenge in developing effective liver-stage vaccines against malaria has been in identifying suitable target antigens to induce strong protective effects.

Murine malaria models exist but these are not helpful for studying *P. falciparum* infection in humans, and many antigens in *P. falciparum* have no homologues in the rodent parasites. Furthermore, hundreds or perhaps thousands of the 5000 or so genes in the *P. falciparum* genome are likely expressed in the liver and there has been no way of finding out which of these is a good vaccine immunogen. However, it is likely that only a small number or minority of the many genes expressed in the liver by *P. falciparum* produce proteins that end up as peptides presented by MHC class I molecules on the infected liver cell surface. These are the potential targets of vaccine-induced T cells whereas antigens that do not reach the surface in MHC molecules cannot be protective when using a liver stage vaccine. Because parasite antigens in the liver are inside a parasitophorous vacuole, which is surrounded by a parasitophorous vacuole (PV) membrane, most parasite antigens will be unable to reach the liver cell cytoplasm where they can be degraded, loaded on the MHC molecules and transported to the hepatocyte surface. Due to various technical difficulties limiting the ability of identification of the MHC- peptide complexes on the liver-cell surface directly, it has not been possible to determine which *P. falciparum* antigens/immunogens would be suitable liver-stage vaccine antigens/immunogens. These difficulties include:
- *in vitro* only a small number of liver cells can be infected by *P. falciparum* and the excess of self-peptides from non-infected and also infected cells makes identification of parasite peptides very difficult, even using state of the art mass spectrometry techniques to sequence eluted peptides;
- direct analysis of infected human livers (where only a tiny proportion of cells is infected) with ongoing malaria liver-stage infection is not practicable;
- separation of parasitized liver cells from non-infected ones has proven difficult in the absence of suitable labelled parasites for use as tools; and
- many hundreds of thousands, millions, or even billions of infected cells are required to allow a reasonable number of infected hepatocytes to be studied.

Further, as it has been possible to achieve higher efficacy in humans by immunizing with whole irradiated sporozoites, it is widely believed that there may well be other, more protective, immunogens yet to be identified that are expressed by the *Plasmodium* parasite inside liver cells, which when used as vaccines would be more suitable and effective.

Therefore, it would be desirable to provide alternative immunogens, and improved delivery and vaccination methods for eliciting a protective immune response against malaria.

The inventors have identified proteins and peptides which are presented on HLA class I molecules of *Plasmodium* infected cells. Surprisingly, a large proportion (approx. 57%) are derived from ribosomal proteins or ribosomal associated proteins of the parasite, many of which are highly conserved between *Plasmodium* species. The peptides have been shown to be effective immunogens capable of producing a protective cellular response against the Plasmodium parasite, and provide a new class of immunogens useful in vaccines, in particular against malaria.

The present invention provides novel immunogens which may be used in vaccine compositions; in particular the invention provides novel immunogens which may be used in vaccine compositions for use against malaria, preferably wherein the vaccine targets the CD8+T cell immunological response, both at liver and blood stage.

According to a first aspect of the invention, there is provided one or more plasmodium-derived proteins or immunogenic fragments thereof for use in eliciting an immune response in a subject. The one or more plasmodium-derived protein or immunogenic fragment thereof may be, or may be derived from, a ribosomal protein or ribosomal associated protein. The one or more plasmodium-derived protein or immunogenic fragment thereof may be, or may be derived from, a malarial early transcribed membrane protein (ETRAMP) or a histone. The one or more plasmodium-derived protein or immunogenic fragment thereof may be, or may be derived from, a protein recited in Figure 1, or a protein or a peptide recited in Figure 2 or Figure 3.

The one or more plasmodium-derived protein or immunogenic fragment thereof may be derived from *Plasmodium falciparum* and/or from *Plasmodium vivax.*

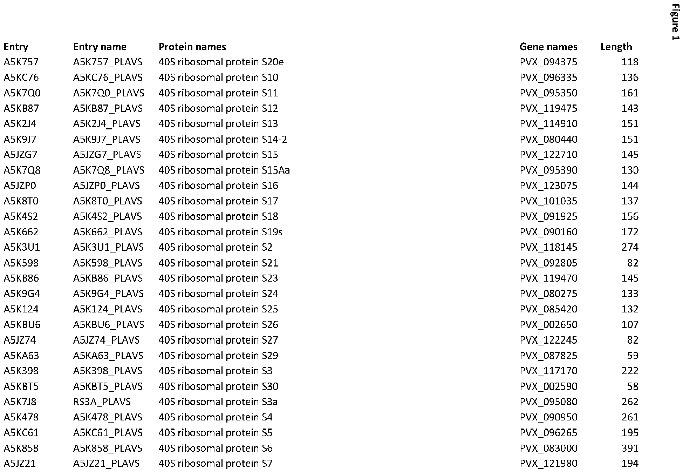

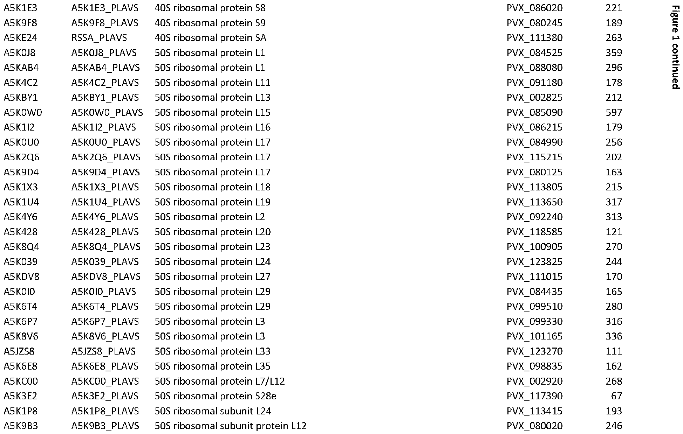

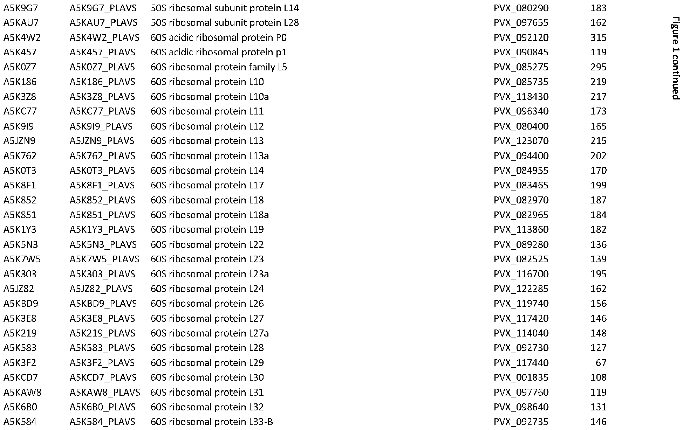

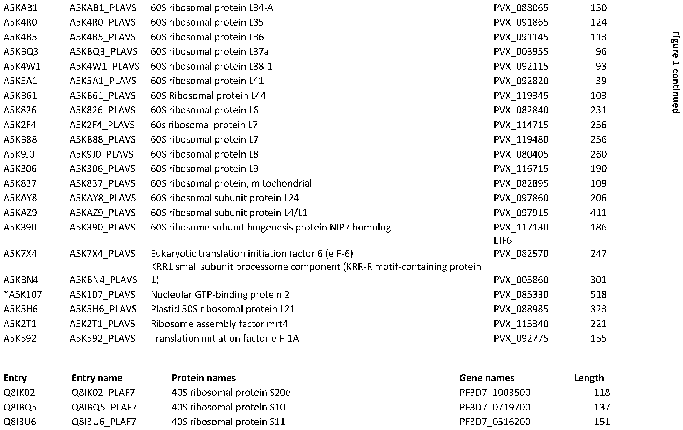

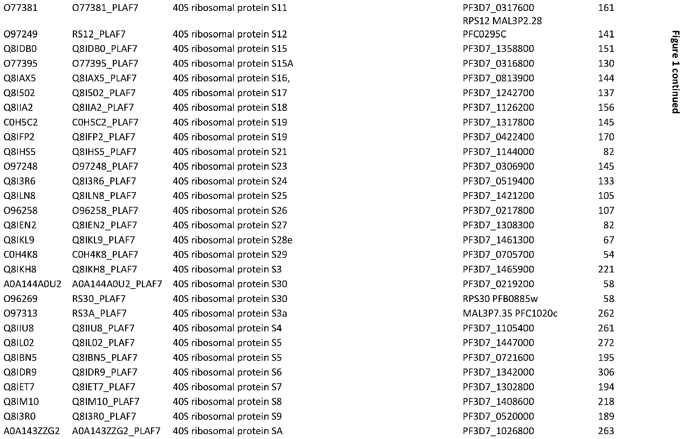

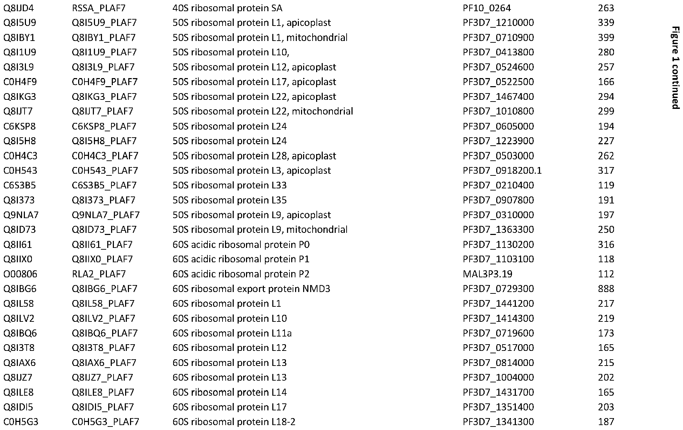

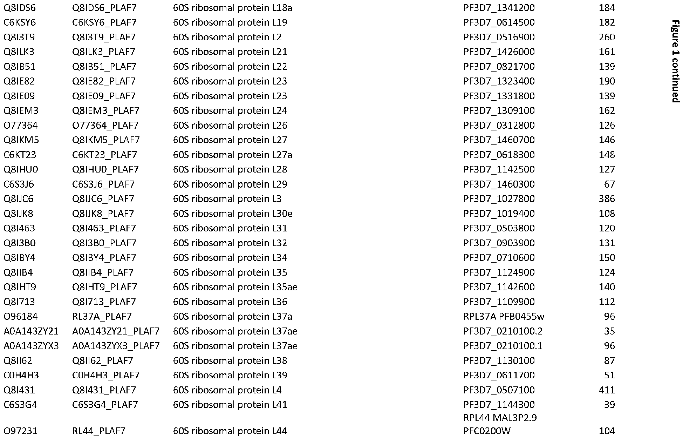

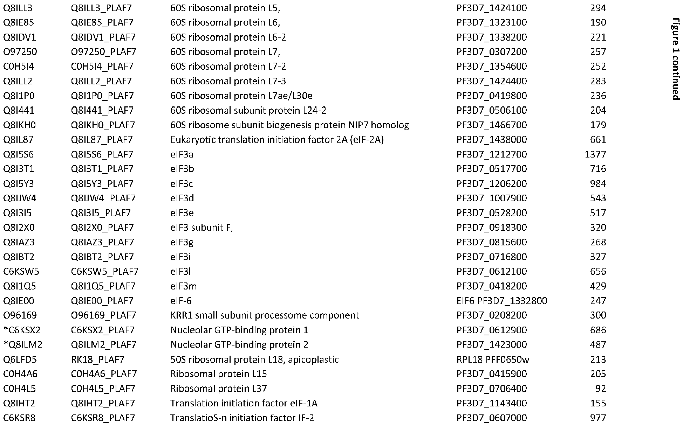

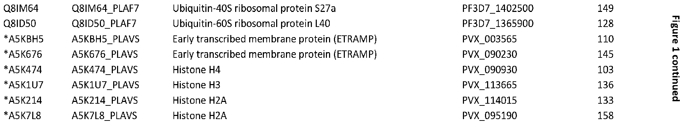

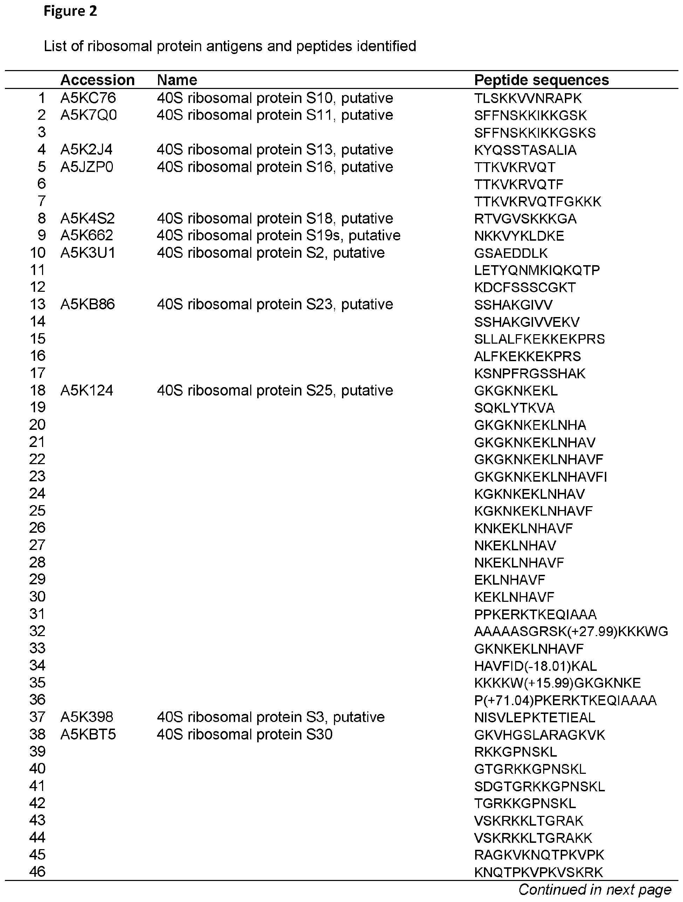

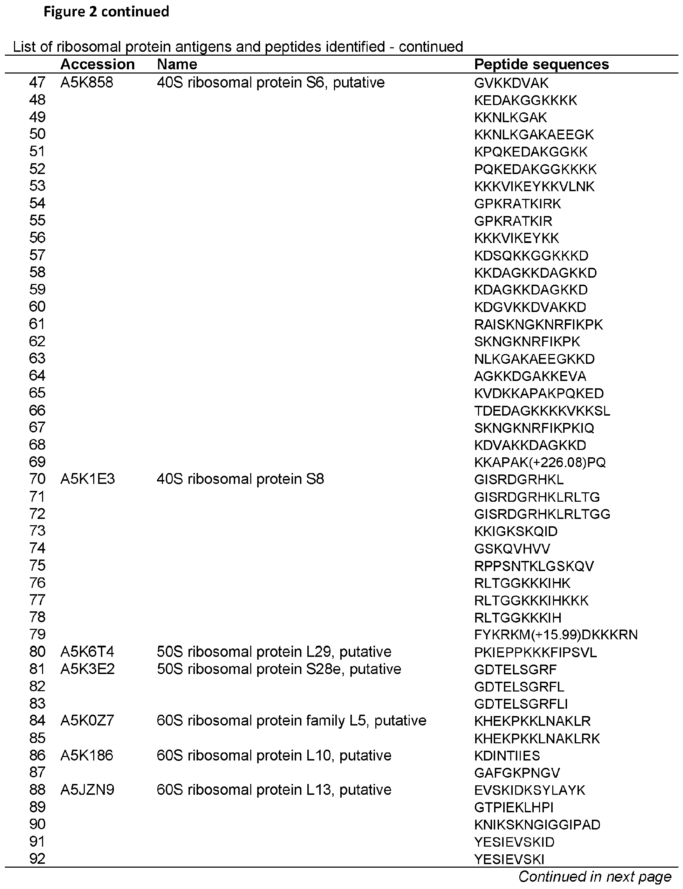

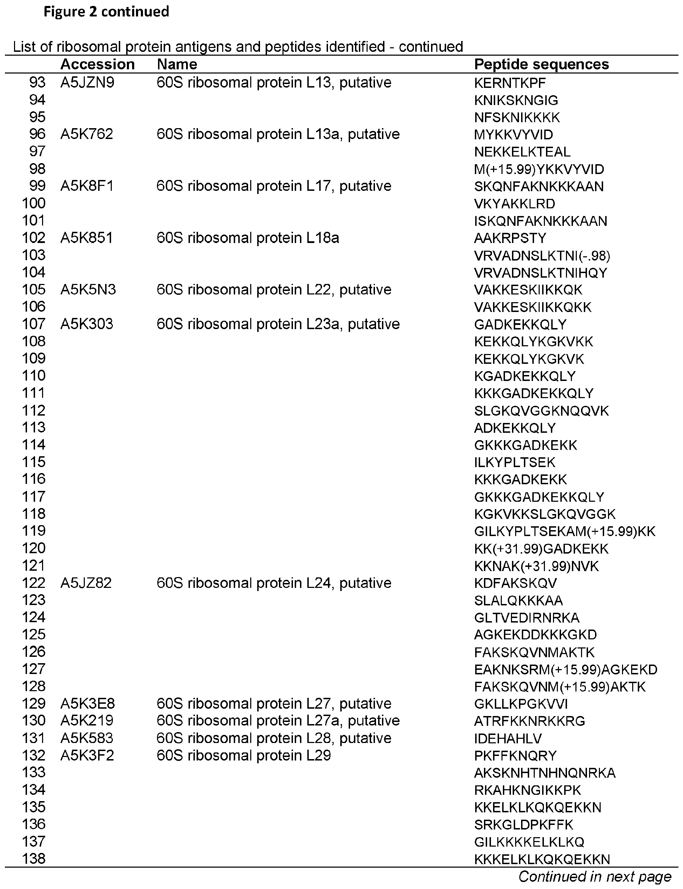

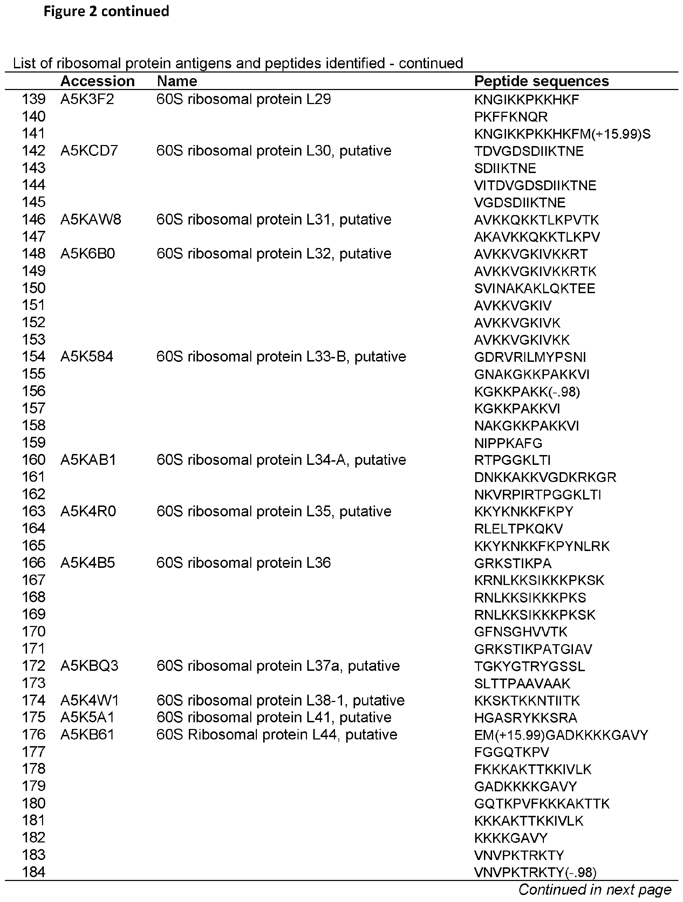

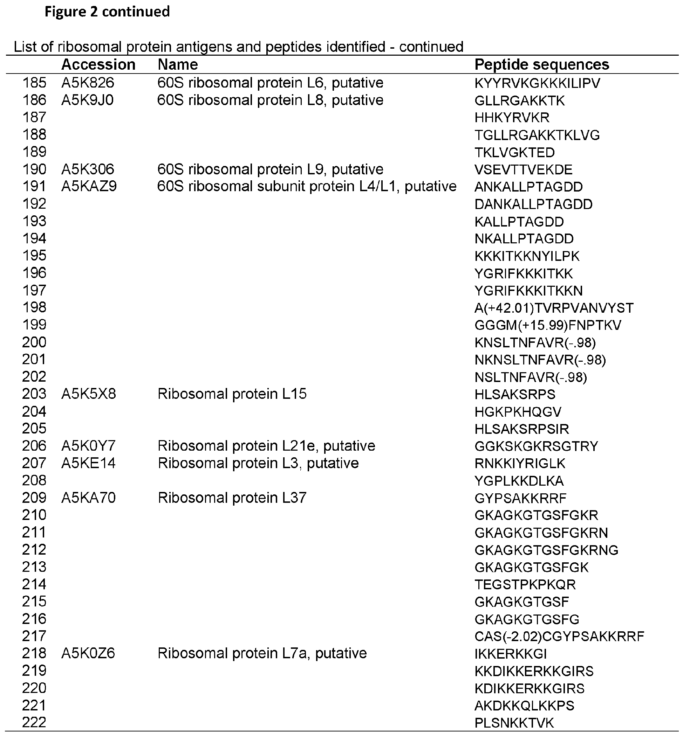

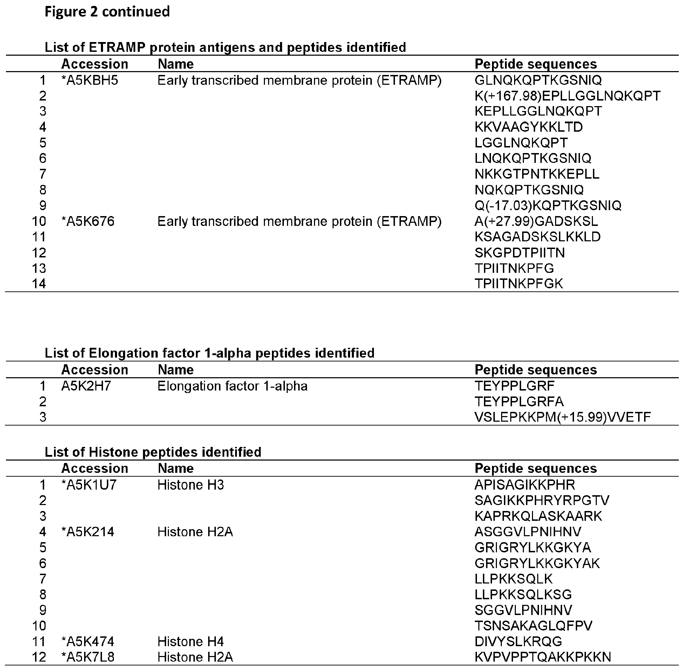

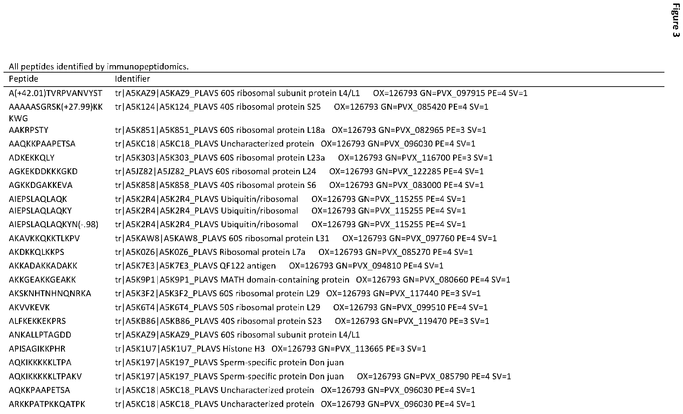

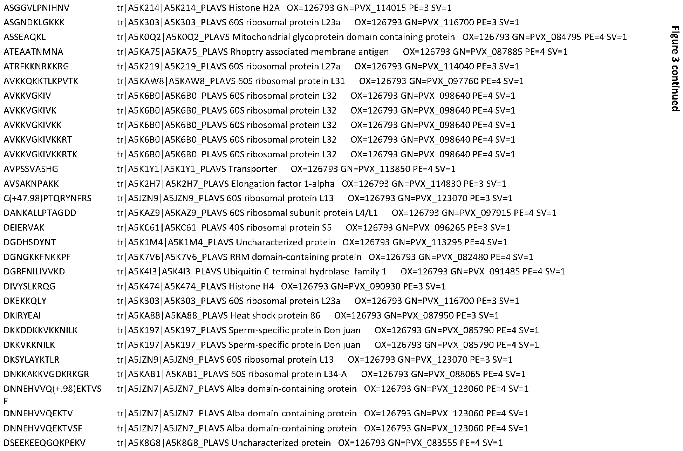

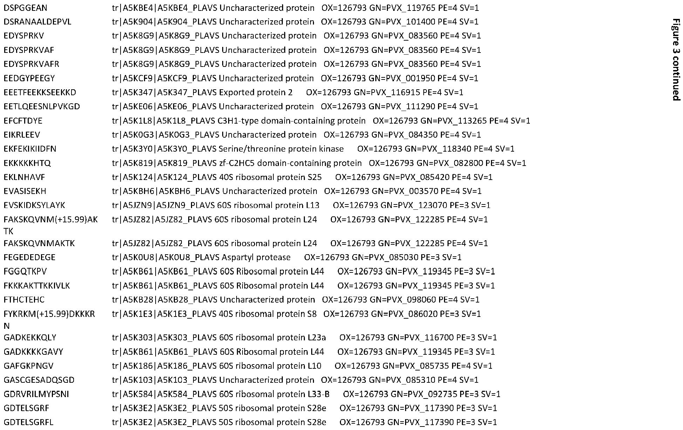

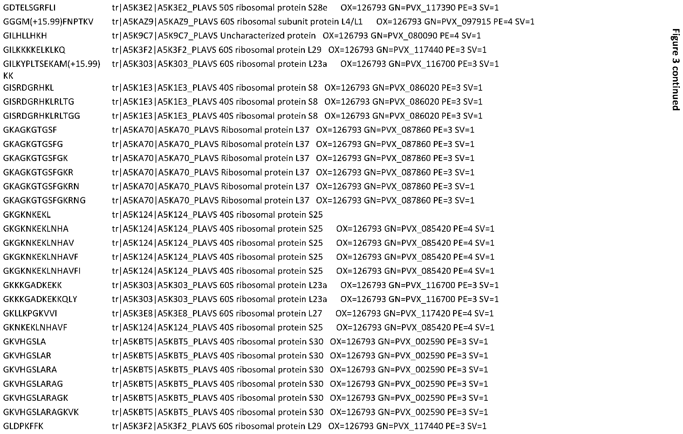

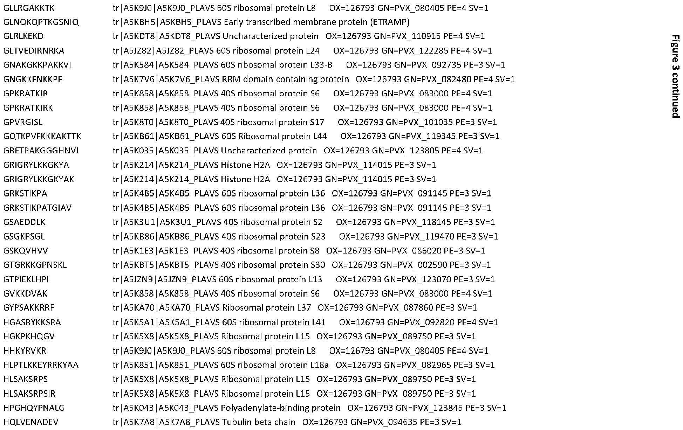

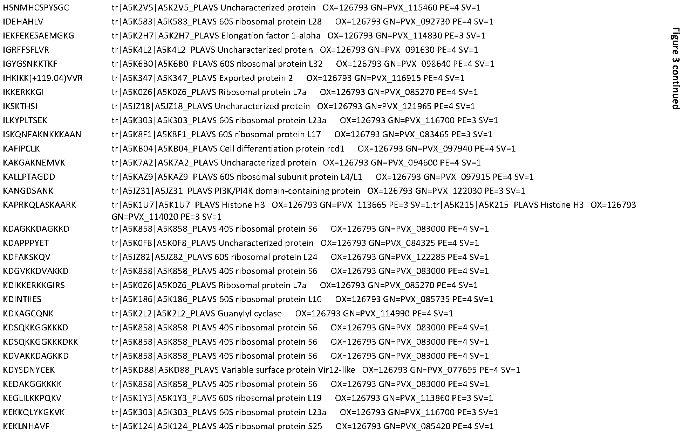

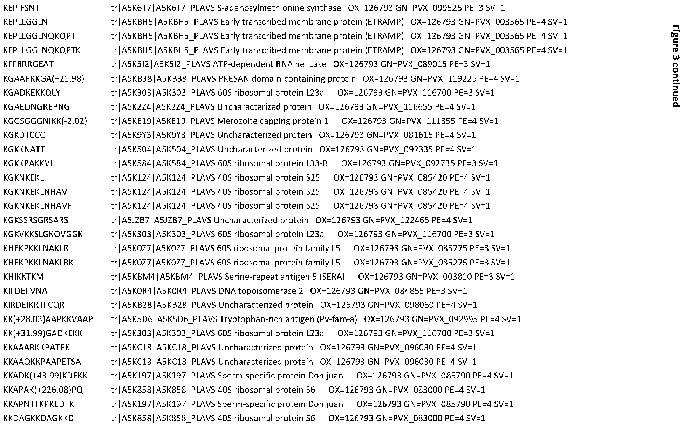

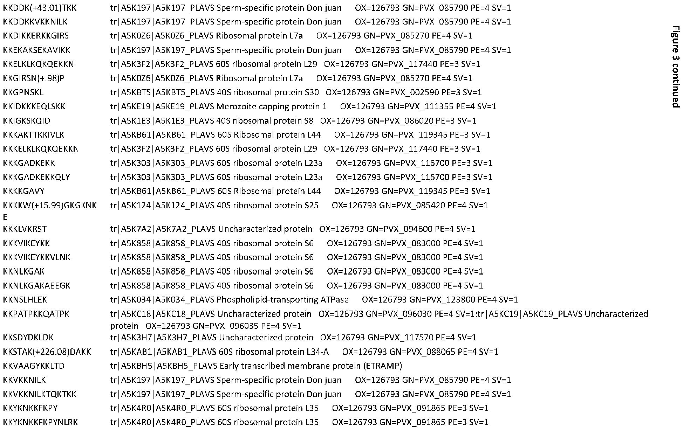

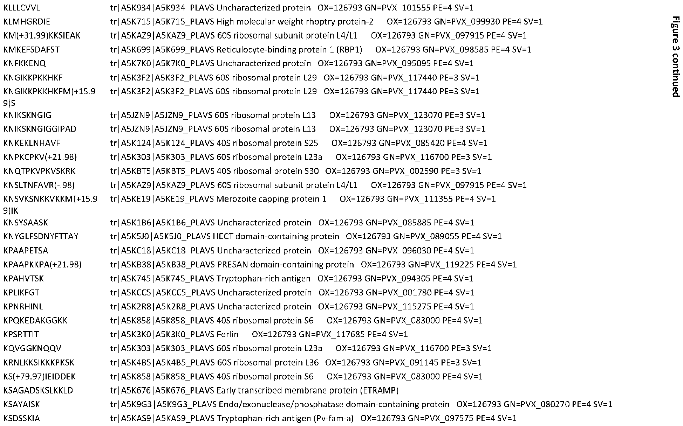

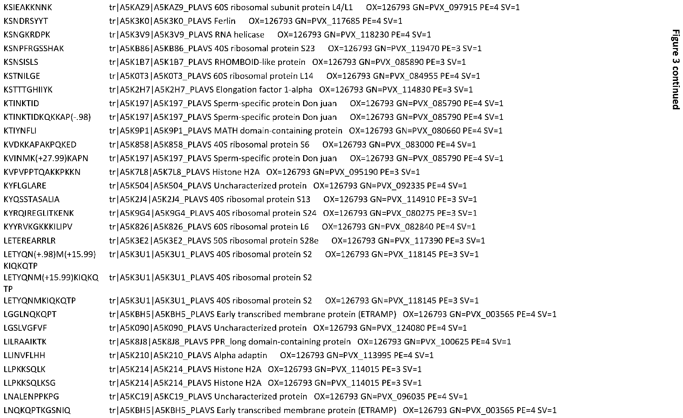

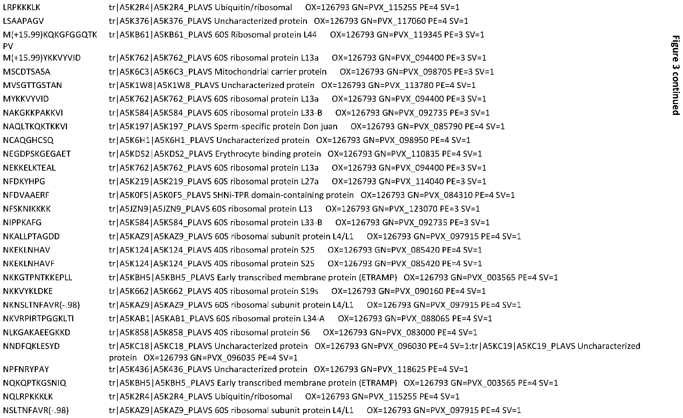

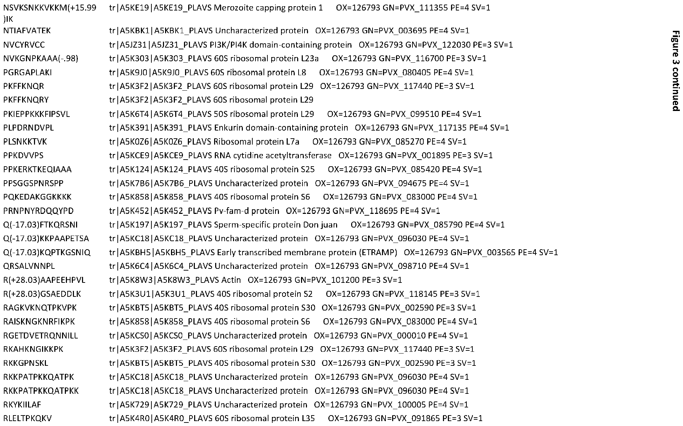

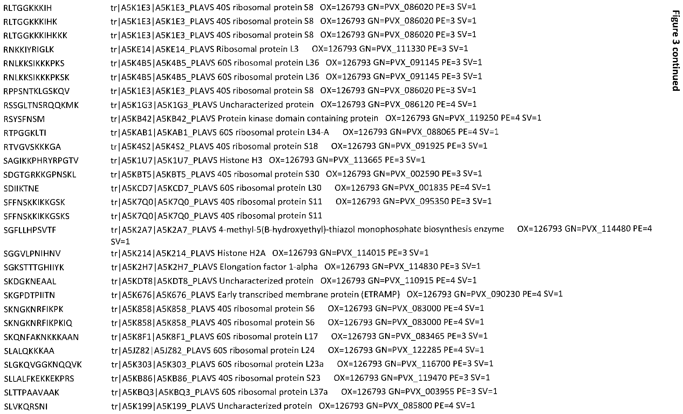

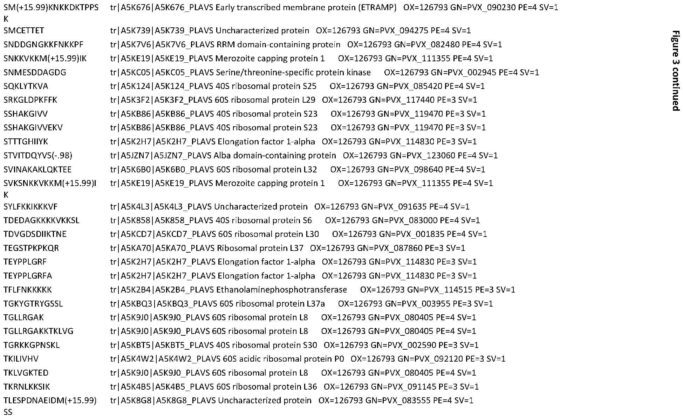

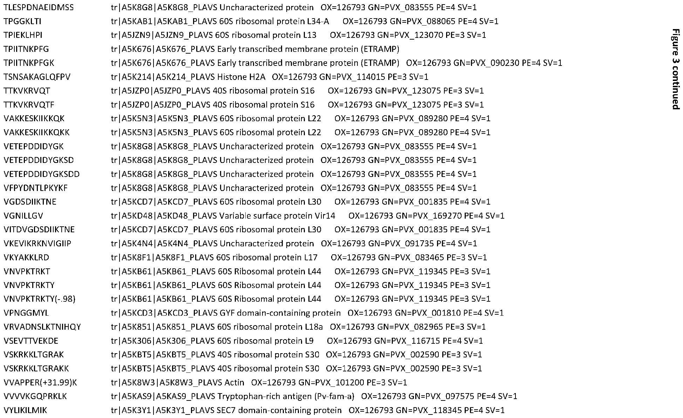

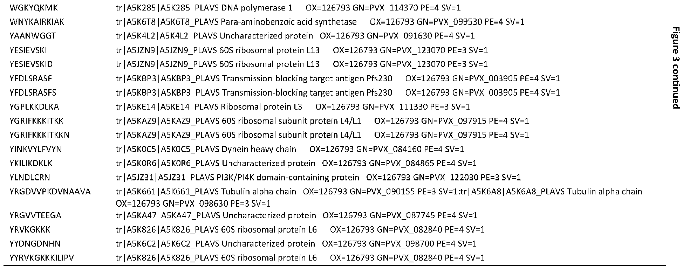

The invention may provide one or more *Plasmodium falciparum* or *Plasmodium vivax* proteins or immunogenic fragments thereof for use in raising an immune response in a subject. The one or more plasmodium-derived protein or immunogenic fragment thereof may be, or may be derived from, a ribosomal protein or ribosome associated protein. The one or more plasmodium-derived protein or immunogenic fragment thereof may be, or may be derived from, a malarial early transcribed membrane protein (ETRAMP) or a histone. The one or more plasmodium-derived protein or immunogenic fragment thereof may be, or may be derived from, a protein recited in Figure 1, or a protein or a peptide recited in Figure 2 or Figure 3. The immune response elicited may be to prevent or treat malaria.

In a further aspect, the invention provides an immunogenic composition comprising:
a) one or more plasmodium-derived protein or immunogenic fragment thereof; and/or
b) a polynucleotide encoding one or more plasmodium-derived protein or immunogenic fragment thereof. The one or more plasmodium-derived protein or immunogenic fragment thereof may be, or may be derived from, a ribosomal protein or ribosomal associated protein. The one or more plasmodium-derived protein or immunogenic fragment thereof may be, or may be derived from, an ETRAMP or a histone. The one or more plasmodium-derived protein or immunogenic fragment thereof may be, or may be derived from, a protein recited in Figure 1, or a protein or a peptide recited in Figure 2 or Figure 3.

The immunogenic composition may further comprise a pharmaceutically acceptable excipient or carrier. The one or more plasmodium-derived protein or immunogenic fragment thereof may be derived from *Plasmodium falciparum* and/or from *Plasmodium vivax.* The immunogenic composition may be used as a vaccine to treat or prevent malaria.

In another aspect the invention provides a method of eliciting an immune response in a subject, the method comprising the step of administering an immunogenic composition according to the invention to the subject. Preferably the immune response elicited is sufficient to treat or prevent malaria.

In a further aspect of the invention, there is provided an immunogenic composition according to the invention for use in treating or preventing malaria.

In a further aspect of the invention, there is provided an immunogenic composition according to the invention for use in the preparation of a medicament for treating or preventing malaria.

The immune response elicited in a subject by a composition according to the invention may be against malaria. Suitably, the immune response elicited may be sufficient to treat or prevent malaria caused by infection with *Plasmodium falciparum* and/or *Plasmodium vivax.* The malaria may comprise liver-stage malaria. The malaria may comprise pre-erythrocytic-stage malaria. The malaria may comprise erythrocytic-stage malaria.

The immune response elicited by a composition of the invention may be a protective immune response. Suitably, the protective immune response induces the activation of CD8+ T-cells. Where the immunogen is or is derived from a ribosomal protein or a ribosomal associated protein, or an ETRAMP, or a histone, or a protein recited in Figure 1, or a protein or a peptide recited in Figure 2 or Figure 3, or a fragment thereof derived from *Plasmodium falciparum* and/or from *Plasmodium vivax,* the protective immune response elicited may treat or prevent malaria, in particular the immune response may activate CD8+ T-cells which may induce cytotoxicity of *Plasmodium falciparum* and/or *Plasmodium vivax* infected hepatocytes and infected reticulocytes.

The term "protective immune response" used herein, may be understood to be a host immune response that can sterilise the *Plasmodium* infection in a subject or reduce the number of parasites emerging from the liver (so that they are more readily cleared by blood-stage immunity). The protective immune response may sterilise the *Plasmodium* infection in at least 20% of subjects treated. The protective immune response may sterilise the *Plasmodium* infection in at least 35% of subjects treated. The protective immune response may sterilise the *Plasmodium* infection in at least 40% of subjects treated. The protective immune response may sterilise the *Plasmodium* infection in at least 50% of subjects treated. The protective immune response may sterilise the *Plasmodium* infection in at least 60% of subjects treated.

The protective immune response may provide clinical benefit in a subject by preventing the development of clinical malaria of a chronic parasitaemia. A protective immune response may comprise at least 0.1% of CD8+ T cells being antigen/immunogen-specific as determined, for example, by flow cytometry staining, and/or at least 500 spot forming cells (SFU) per million peripheral blood mononuclear cells (PBMC). Spot forming cells (SFU) may be determined by an ELISpot assay (enzyme-linked immunosorbent spot assay (For example the ELISpot assay provided by Mabtech AB, Sweden, see: http://www.mabtech. com/Main/Page. asp?PageId= 16).

A protective immune response may comprise at least 0.2% of CD8+ T cells being antigen/immunogen-specific. A protective immune response may comprise at least 0.4% of CD8+ T cells being antigen/immunogen-specific. A protective immune response may comprise at least 0.8% of CD8+ T cells being antigen/immunogen-specific. A protective immune response may comprise at least 1% of CD8+ T cells being antigen/immunogen-specific.

A protective immune response may comprise at least 100 spot forming cells (SFU) per million peripheral blood mononuclear cells (PBMC). A protective immune response may comprise at least 300 spot forming cells (SFU) per million peripheral blood mononuclear cells (PBMC). A protective immune response may comprise at least 1000 spot forming cells (SFU) per million peripheral blood mononuclear cells (PBMC). A protective immune response may comprise at least 2000 spot forming cells (SFU) per million peripheral blood mononuclear cells (PBMC).

The plasmodium-derived protein or immunogenic fragment thereof may have a sequence which is 80%, 85%, 90%, 95%, 98%, 99% or 100% identical to a protein or a fragment of a protein recited in Figure 1, or a peptide recited in Figure 2 or Figure 3.

The plasmodium-derived protein or immunogenic fragment thereof may have a sequence which is 80%, 85%, 90%, 95%, 98%, 99% or 100% identical to a protein or a fragment of a protein recited in Figure 2 or Figure 3.

The plasmodium-derived protein or immunogenic fragment thereof may have a sequence which is 80%, 85%, 90%, 95%, 98%, 99% or 100% identical to a 40S ribosomal protein, preferably the 40S ribosomal protein S20e or a fragment of the 40S ribosomal protein S20e. The 40S ribosomal protein S20e may be derived from *P. falciparum* (Accession Q8IK02) or *P*. *Vivax* (Accession A5K757). Preferably, the 40S ribosomal protein S20e is derived from *P. falciparum* (Accession Q8IK02).

The plasmodium-derived protein or immunogenic fragment thereof may have a sequence which is 80%, 85%, 90%, 95%, 98%, 99% or 100% identical to an ETRAMP or a fragment of an ETRAMP. The ETRAMP may be derived from *P. falciparum* or *P*. *Vivax.* Preferably, the ETRAMP is derived from *P*. *vivax* (Accession A5KBH5 and A5K676).

The plasmodium-derived protein or immunogenic fragment thereof may have a sequence which is 80%, 85%, 90%, 95%, 98%, 99% or 100% identical to a protein or a fragment of a protein selected from:
- the 40S ribosomal protein S20e (Accession A5K757)
- 60S ribosomal subunit protein L4/L1, putative (Accession A5KAZ9)
- 40S ribosomal protein S25, putative (Accession A5K124)
- Early transcribed membrane protein (ETRAMP) (Accession A5K676)
- Early transcribed membrane protein (ETRAMP) (Accession A5KBH5)
- 40S ribosomal protein S2, putative (Accession A5K3U1)
- 60S ribosomal protein L9, putative (Accession A5K306)
- 60S ribosomal protein L30, putative (Accession A5KCD7)
- 40S ribosomal protein S23, putative (Accession A5KB86)
- 40S ribosomal protein S30 (Accession A5KBT5)
- 60S ribosomal protein L29 (Accession A5K3F2)
- 40S ribosomal protein S6, putative (Accession A5K858)
- 40S ribosomal protein S8 (Accession A5K1E3)
- 60S ribosomal protein L13, putative (Accession A5JZN9)
- 60S ribosomal protein L23a, putative (Accession A5K303)
- Ribosomal protein L37 (Accession A5KA70)
- 40S ribosomal protein S 11, putative (Accession A5K7Q0)
- Histone H2A (Accession A5K214)
- Histone H3 (Accession A5K1U7).

Accession numbers, where given, relate to those identifiable using UniProt (Universal Protein Resource), a comprehensive catalogue of information on proteins ('UniProt: a hub for protein information' Nucleic Acids Res. 43: D204-D212 (2015)).

Due to a high level of sequence conservation across the plasmodium species, a high level of sequence homology exists between many plasmodium-derived proteins, including ribosomal proteins and ribosomal associated proteins, of *Plasmodium falciparum* and *Plasmodium vivax.* A plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof, derived from either *Plasmodium falciparum* or *Plasmodium vivax* may therefore be cross-reactive in raising an immune response in a subject. Suitably, a plasmodium-derived protein or immunogenic fragment thereof, such as a ribosomal protein or ribosomal associated protein or an ETRAMP, or a histone, or a protein or peptide of Figure 1, Figure 2 or Figure 3, or an immunogenic fragment thereof derived from *Plasmodium falciparum* may be suitable for use in eliciting an immune response to treat or prevent malaria in a subject infected with either *Plasmodium falciparum* or *Plasmodium vivax.* Suitably, a plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof derived from *Plasmodium vivax* may be suitable for use in eliciting an immune response to treat or prevent malaria in a subject infected with either *Plasmodium falciparum* or *Plasmodium vivax.*

The term "fragment" encompasses immunogenic and/or antigenic fragments of a plasmodium-derived protein or immunogenic fragment thereof, such as a ribosomal protein or ribosomal associated protein, or an ETRAMP, or a histone, or a protein or peptide of Figure 1, Figure 2 or Figure 3. Preferably the fragments elicit an immune response when administered to a subject sufficient to generate a protective immune response. The fragment may be derived from a ribosomal protein or ribosomal associated protein, or an ETRAMP, or histone, or a protein or peptide of Figure 1, Figure 2 or Figure 3, from *Plasmodium falciparum* and/or from *Plasmodium vivax,* and the protective immune response elicited may be sufficient to treat or prevent malaria. The fragment is preferably from about 5 to about 20 amino acids long, preferably from about 7 to about 15 amino acids long. The fragment may have a sequence which is 80%, 85%, 90%, 95%, 98%, 99% or 100% identical to a peptide recited in Figure 2 or Figure 3.

Percentage sequence identity is defined as the percentage of amino acids in a sequence that are identical with the amino acids in a provided sequence after aligning the sequences and introducing gaps if necessary to achieve the maximum percent sequence identity. Alignment for the purpose of determining percent sequence identity can be achieved in many ways that are well known to the man skilled in the art, and include, for example, using BLAST (National Center for Biotechnology Information Basic Local Alignment Search Tool).

Variations in percent identity may be due, for example, to amino acid substitutions, insertions or deletions.

The plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof may include one or more conservative amino acid substitutions - the term "conservative substitution" embracing the act of replacing one or more amino acids of a protein or peptide with an alternate amino acid with similar properties and which does not substantially alter the physiochemical properties and/or structure or function of the native (or wild type) protein, for example the substitution of leucine with isoleucine is a conservative substitution. Other conservative substitutions include: Ala → Gly, Ser, Val; Arg → Lys; Asn → Gin, His; Asp → Glu; Cys → Ser; Gin → Asn; Glu → Asp; Gly → Ala; His → Arg, Asn, Gln; Ile → Leu, Val; Leu → Ile, Val; Lys → Arg, Gln, Glu; Met → Leu, Tyr, He; Phe → Met, Leu, Tyr; Ser → Thr; Thr → Ser; Trp → Tyr; Tyr → Trp, Phe; Val → He, Leu. Other substitutions are also permissible and can be determined empirically or in accord with other known conservative or non-conservative substitutions.

The skilled person will be readily able to determine the polynucleotide sequence that would be needed to encode the immunogen. Of course, one of skill will appreciate that the degeneracy of the genetic code permits substitution of one or more bases in a codon without changing the primary amino acid sequence encoded. The skilled person will also appreciate the existence of codon bias and may tailor any polynucleotide sequence to the organism in which it will be expressed, preferably a human.

A polynucleotide encoding the plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof may be codon optimised. The codon optimisation may be for optimal translation in a mammalian host cell, such as a human host cell.

The polynucleotide in the immunogenic composition may be provided in a vector. The vector may be a viral vector. Alternatively the polynucleotide may be provided in a plasmid DNA vector or as an RNA vector-based immunogen.

The polynucleotide may be expressed as a protein in a variety of cells that are known in the field (Pichia, human cell lines, simian cell lines, insect cell lines, bacterial host etc.). The expressed protein encoded by the polynucleotide may be delivered as a vaccine, typically in combination with an adjuvant. Adjuvants and adjuvant formulations are well known, such as alum, AS01, matrix-M, MF59, GLA, Hiltonol and others.

In an aspect, the invention may provide a vector comprising a polynucleotide encoding one or more plasmodium-derived protein or immunogenic fragment thereof, such as a ribosomal protein or ribosomal associated protein, or an ETRAMP, or a histone or a protein or peptide of Figure 1, Figure 2 or Figure 3, or an immunogenic fragment thereof as defined herein. The vector may be a live vector, such as a viral vector or a eukaryotic vector. The vector may be a viral vector, a DNA vector or an RNA vector.

In a further aspect the invention may provide an immunogenic composition comprising a vector of the invention, and optionally a pharmaceutically acceptable carrier or excipient. The vector may be a viral vector.

In a yet further aspect the invention may provide an immunogenic composition comprising a vector according to the invention and a pharmaceutically acceptable carrier for use as a vaccine.

The vector may be a virus (viral vector) or a protozoa parasite capable of delivering a polynucleotide encoding one or more plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof into a host cell, such as a mammalian host cell, for example a liver cell such as a hepatocyte. The genetic material may be heterologous nucleic acid, which is not naturally encoded by the vector and/or the host cell. The vector may be modified by mutation to reduce its pathogenicity. The vector may be modified to encode an immunogenic protein or fragment thereof, in particular one or more ribosomal protein or ribosomal associated protein, or an ETRAMP, or a histone, or a protein or peptide of Figure 1, Figure 2 or Figure 3, or an immunogenic fragment thereof according to the invention. The vector may be a viral vector and may comprise an adenovirus. Suitably, the vector is configured to express one or more immunogens, for example one or more plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof according to the invention.

The viral vector may comprise a Modified Vaccinia Ankara (MVA) virus. The viral vector may be selected from any of the group comprising, a poxvirus, such as Modified Vaccinia Ankara (MVA) virus, or an adenovirus. The adenovirus may comprise a simian or human adenovirus. The adenovirus may comprise a Group E adenovirus. The adenovirus may comprise ChAd63 or ChAd3 or ChAdOx1or ChAdOx2 or gorilla-derived adenoviruses. The adenovirus may comprise ChAdOxl. The adenovirus may comprise a group A, B, C , D or E adenovirus. The adenovirus may comprise Ad35, Ad5, Ad6, Ad26, or Ad28. The adenovirus may be of simian (e.g. chimpanzee, gorilla or bonobo) origin. The adenovirus may comprise any of ChAd63, ChAdOxl, ChAdOx2, C6, C7, C9, PanAd3, or ChAd3. The protozoa vector may comprise a *Trypanosoma cruzi.* The viral vector may be selected from any of the group comprising, a Trypanosomatidae, such as *Trypanosoma cruzi,* or *Leishmania.* The *Trypanosoma cruzi* may comprise *Trypanosoma cruzi* CL-14. The composition may comprise two or more different vectors. One or more of the vectors may be live vectors. One or more of the vectors may be viral vectors.

The plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof may be administered to a subject as a vaccine in a single dose vaccination regime or as a multidose regimen, for example a prime-boost vaccination regime, or a prime-target vaccination regime.

In a single dose vaccination regime the plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof, may be administered as a protein/peptide or via a polynucleotide which enables the one or more plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof to be expressed in a host subject after administration. Where the one or more plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof is administered as a polynucleotide for expression, the polynucleotide may be provided in a vector as described herein. The vector may be a live vector. The vector may be a viral vector. The administration may be a single dose vaccination regime using just the adenoviral vector, or the MVA vector, or a mixture of both. The administration may be a single dose vaccination regime using just *Trypanosoma cruzi.*

The administration may be part of a prime-boost vaccination regime in a subject, where a first/prime administration of the immunogenic composition of the invention is followed by a second/boost administration of the immunogenic composition of the invention.

The administration may be part of a prime-target vaccination regime in a subject, where a first/prime administration of the immunogenic composition of the invention is followed by a second/boost administration of the immunogenic composition of the invention, wherein the boost dose is administered by an intravenous route or other route that leads to antigen deposition in the liver.

Additional boost vaccinations may be provided. Alternatively only one of the prime and boosts may comprise a composition of the invention. Alternatively, only a single dose of the vaccine may be required to induce protective immunity.

Where the immunogenic composition is intended for a multiple administration regime, such as a prime-boost regime or prime-target regime, the different administration may comprise identical or different immunogenic compositions or vaccines or pharmaceutical compositions. Where the immunogenic composition is intended for a prime-boost or prime-target administration regime, the prime composition may comprise the same or different viral vector as the boost composition. The same immunogenic composition may be used for both prime and boost administrations. A different immunogenic composition or vaccine may be used for the prime and boost administrations.

The viral vector of the first/prime administration may comprise adenovirus. The viral vector of the second/boost administration may comprise poxvirus, such as MVA, or adenovirus.

The second/boost administration may be between about 1 day and about 30 days after the first/prime administration. The second/boost administration may be about 14 days after the first/prime administration.

Additional administrations of the immunogenic composition of the invention may be provided.

Suitable doses of adenoviral vectors for immunising humans are about 1 × 10⁸ to about 1 × 10¹¹ viral particles. Suitable doses of MVA for immunising humans are about 1 × 10⁷ pfu to about 1 × 10⁹ pfu. These vectors may be given by a range of immunisation routes, typically intramuscular and intravenous, but also for example subcutaneous, intradermal, intranasal and aerosol.

According to a yet further aspect, the invention provides a host cell comprising a vector, preferably a viral vector, according to the invention. The host cell may be *in vitro.* The host cell may be infected with the viral vector of the invention, or may comprise and express a polynucleotide of the invention.

Suitable vectors can be chosen or constructed, containing appropriate regulatory sequences, including promoter sequences, terminator sequences, polyadenylation sequences, enhancer sequences, invariant chain sequences, marker genes and other sequences as appropriate. For further details see, for example, (Sambrook, J., E. F. Fritsch, and T. Maniatis. (1989), Molecular cloning: a laboratory manual, 2nd ed. Cold Spring Harbor Laboratory, Cold Spring Harbor, New York). Many known techniques and protocols for manipulation of nucleic acid, for example in preparation of nucleic acid constructs, mutagenesis, sequencing, introduction of DNA into cells and gene expression, and analysis of proteins, are described in detail in (Ausubel et al., Current protocols in molecular biology. New York: Greene Publishing Association; Wiley-Interscience, 1992). A polynucleotide of the invention may be expressed using any suitable expression system, for example in a suitable host cell infected with a viral vector encoding a polynucleotide of the invention.

A composition of the invention can be formulated using established methods of preparation (Gennaro, A.L. and Gennaro, A.R. (2000) Remington: The Science and Practice of Pharmacy, 20th Ed., Lippincott Williams & Wilkins, Philadelphia, PA).

A composition of the invention may be administered via any parenteral or non-parenteral (enteral) route that is therapeutically effective. Parenteral application methods include, for example, intracutaneous, subcutaneous, intramuscular, intratracheal, intranasal, intravitreal or intravenous injection and infusion techniques, e.g. in the form of injection solutions, infusion solutions or mixtures, as well as aerosol installation and inhalation, e.g. in the form of aerosol mixtures, sprays or powders. In a preferred embodiment, a composition of the invention is administered intramuscularly or intravenously. A composition can be administered systemically in a formulation containing conventional non-toxic pharmaceutically acceptable excipients or carriers, additives and vehicles as desired. The composition may be an aqueous solution, an oil-in water emulsion or a water-in-oil emulsion.

To prepare the compositions, pharmaceutically inert inorganic or organic excipients can be used. To prepare for example pills, powders, gelatin capsules or suppositories, lactose, talc, stearic acid and its salts, fats, waxes, solid or liquid polyols, natural and hardened oils are examples of pharmaceutically acceptable excipients which can be used. Suitable excipients for the production of solutions, suspensions, emulsions, aerosol mixtures or powders for reconstitution into solutions or aerosol mixtures prior to use include water, alcohols, glycerol, polyols, and suitable mixtures thereof as well as vegetable oils.

For intravenous injection, the active ingredient will be in the form of a parenterally acceptable aqueous solution which is pyrogen-free and has suitable pH, isotonicity and stability. Those of relevant skill in the art are well able to prepare suitable solutions using for example, isotonic vehicles such as Sodium Chloride Injection, Ringer's Injection, Lactated Ringer's Injection. Preservatives, stabilisers, buffers, antioxidants and/or other additives may be included, as required.

The compositions are preferably administered to an individual in a "therapeutically effective amount", this being sufficient to show benefit to the individual. The optimal dosage will depend on, for example, the biodistribution of the active agent which induces immunogenicity, and the mode of administration.

The composition may also contain additives, such as, for example, fillers, binders, wetting agents, glidants, stabilizers, preservatives, emulsifiers, and furthermore solvents or solubilizers.

A pharmaceutically acceptable carrier can contain physiologically acceptable agents that act, for example, to stabilize, increase solubility or to increase the absorption of a composition. Such physiologically acceptable agents include, for example, carbohydrates, such as glucose, sucrose or dextrans, antioxidants, such as ascorbic acid or glutathione, chelating agents, low molecular weight proteins or other stabilizers or excipients. The choice of a pharmaceutically acceptable carrier, including a physiologically acceptable agent, depends, for example, on the route of administration of the composition. The compositions can be a self-emulsifying drug delivery system or a selfmicroemulsifying drug delivery system. The compositions can also be a liposome or other polymer matrix, which can have incorporated therein, for example, the compositions of the invention. Liposomes, for example, which comprise phospholipids or other lipids, are nontoxic, physiologically acceptable and metabolisable carriers that are relatively simple to make and administer.

The phrase "pharmaceutically acceptable" is employed herein to refer to those compounds, materials, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

A composition of the invention may further comprise, or be intended to be administered simultaneously, sequentially, or separately with an adjuvant. The adjuvant may comprise an oil emulsion. The adjuvant may be selected from any of the group comprising PEI; Alum; AS01 or AS02 (GlaxoSmithKline); inorganic compounds, such as aluminium hydroxide, aluminium phosphate, calcium phosphate hydroxide, or beryllium; mineral oil, such as paraffin oil; emulsions, such as MF59; bacterial products, such as killed bacteria Bordetella pertussis, or Mycobacterium bovis; toxoids; non-bacterial organics, such as squalene or thimerosal; the saponin adjuvant matrix M (Isconova / Novavax) or other ISCOM-type adjuvants; detergents, such as Quil A; cytokines, such as IL-1, IL-2, or IL-12; Freund's complete adjuvant; and Freund's incomplete adjuvant; or combinations thereof.

In another aspect, there is provided a method of treatment or prevention of malaria, said method comprising administering to a subject at risk of or suffering from malaria one or more plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof of the invention; one or more a polynucleotides encoding one or more plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof according to the invention; one or more vectors according to the invention; or one or more immunogenic compositions of the invention.

In another aspect, there is provided one or more plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof, vectors, or immunogenic compositions of the invention for use in the manufacture of a medicament for the treatment or prevention of malaria.

Thus, in another aspect of the invention, there is provided a method of prevention or treatment of malaria in a subject, comprising:
- a first administration of an immunogenic composition of the invention; and
- a second administration of an immunogenic composition of the invention.

In another aspect, there is provided a kit for a vaccination regime against malaria in a subject, comprising:
- a prime composition comprising a viral vector comprising nucleic acid encoding a plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof; and
- a boost composition comprising the same or a different viral vector comprising nucleic acid encoding the same or a different plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof.

The kit may further comprise directions to administer the prime composition prior to the boost composition in a subject. The nucleic acid of the viral vector of the kit may further encode one or more other plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof. The one or more other plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof may comprise *Plasmodium* immunogens capable of eliciting an immune response in a subject.

The kit, prime, and/or boost composition may further comprise an adjuvant.

According to another aspect of the invention, there is provided a method of manufacturing a viral vector of the invention, comprising:
- culturing host cells capable of facilitating viral replication;
- infecting the host cells with a viral vector of the invention, or transfecting host cells with DNA encoding a plasmodium-derived protein or immunogenic fragment thereof, such as a ribosomal protein or ribosomal associated protein, or an ETRAMP, or a histone, or a protein or peptide of Figure 1, Figure 2 or Figure 3, or an immunogenic fragment thereof, or viral vector, of the invention;
- incubating the host cells to allow the production of viral progeny; and
- harvesting and purifying the viral progeny to produce the immunogenic composition.

Suitable cell lines for production of adenoviral vectors include HEK293 cells and Per.C6 cells. Suitable cells for production of MVA include chicken embryo fibroblast, DF1 cells, AGE1.CR.pIX and EB66.

The skilled person will understand that optional features of one embodiment or aspect of the invention may be applicable, where appropriate, to other embodiments or aspects of the invention.

There now follows by way of example only a detailed description of the present invention with reference to the accompanying drawings, in which;
**Figure 1** - is a table of plasmodium-derived proteins, including ribosomal proteins or ribosomal associated proteins, and ETRAMPs, and histones, and other non-ribosomal associated proteins which are marked with an asterix (*) derived from *P. falciparum* and *P. vivax.*
   peptides identified the majority come from plasmodial ribosomal proteins. Non-ribosomal associated proteins are marked with an asterix (*)
**Figure 3** - is a table of all the fragments of plasmodium-derived identified after elution from HLA class I molecules of *P. vivax*-infected reticulocytes and immunopeptidomic mass spectrometry sequence analysis. Of all the peptides identified the majority come from plasmodial ribosomal proteins.

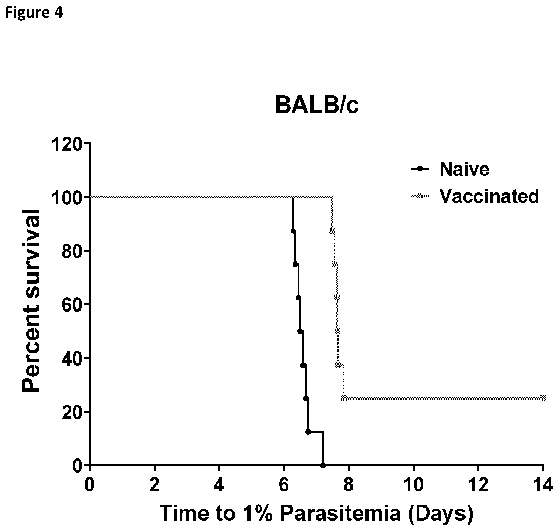

**Figure 4** - demonstrates the protective efficacy of Ad-MVA encoding PfS20e ribosomal protein in immunized mice after challenge with PfS20e- expressing sporozoites transgenic for this gene, expressed using the Pb uis4 promoter. Kaplan-Meier analysis of the survival curves showed a highly significant protective effect with 2/8 mice sterilely protected with a P value of P = 0.0001.

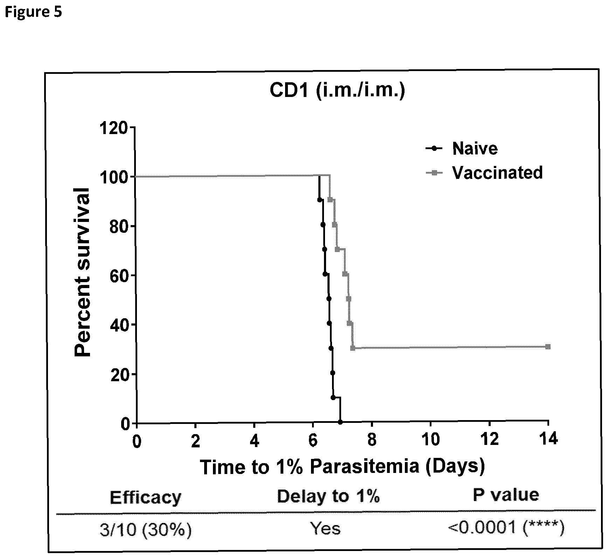

**Figure 5** - demonstrates the protective efficacy of the ChAdOx1-MVA *P. falciparum* S20e vaccine in CD-1 outbred mice (n=10 vaccinated and 10 naive). The mice were vaccinated i.m. with 108 ifu of PfS20e-ChAdOx1 as a prime vaccination, followed eight weeks later by 107 pfu of PfS20e-MVA as a boost vaccination (by i.m. route). Mice were challenged with 1000 chimeric sporozoites i.v. The Kaplan-Meier curves illustrate the time to 1% parasitaemia, whilst statistical significance between the survival curves was assessed using the Log-Rank (Mantel-Cox) Test: p<0.0001.

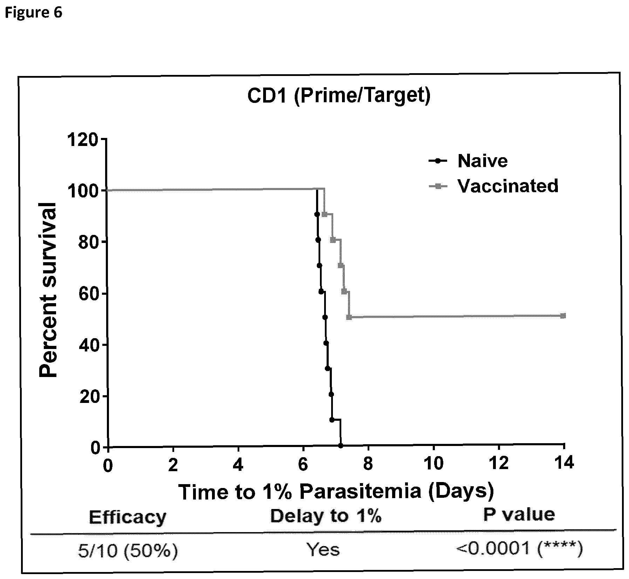

**Figure 6** - shows the protective efficacy of the ChAdOx1-MVA *P. falciparum* S20e vaccine in CD-1 inbred mice (n=10 vaccinated and 10 naive). The mice were vaccinated i.m. with 10⁸ ifu of PfS20e-ChAdOx1 as prime followed three weeks later by 10⁷ pfu of PfS20e-MVA as a target-boost vaccination (by i.v. route). Mice were challenged with 1000 chimeric sporozoites i.v. The Kaplan-Meier curves illustrate the time to 1% parasitaemia, whilst statistical significance between the survival curves was assessed using the Log-Rank (Mantel-Cox) Test: p<0.0001.

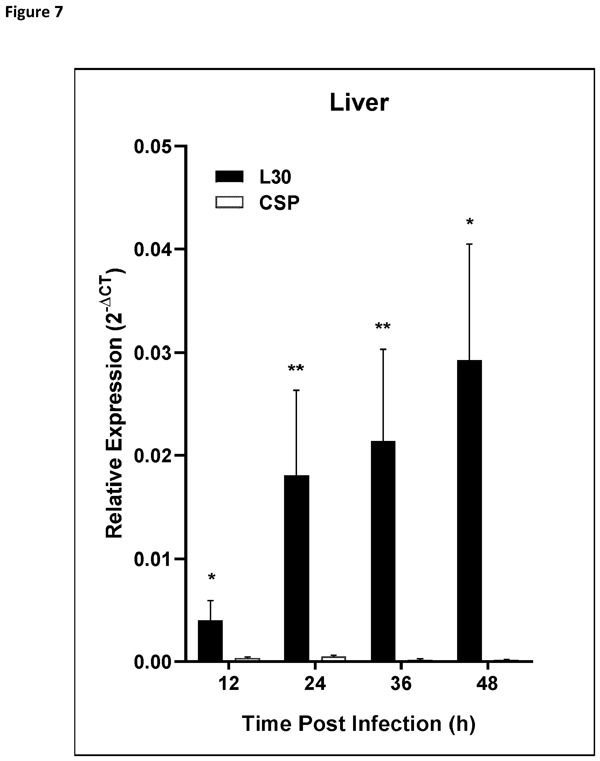

**Figure 7** - demonstrates relative expression of the 60S ribosomal protein L30 *P. berghei* homologue compared to CSP in *P. berghei-infected* hepatocytes at 12, 24, 36 and 48 hours post infection by RT-qPCR normalised to levels of 18SrRNA. Average 2-ΔCT was calculated from two biological replicates, which had three technical replicates each. Error bars show the standard deviation for each sample. Kruskal Wallis Multiple Comparison Dunn's test compared to the relative expression of CSP. ^{∗}P<0.05, **P<0.01.

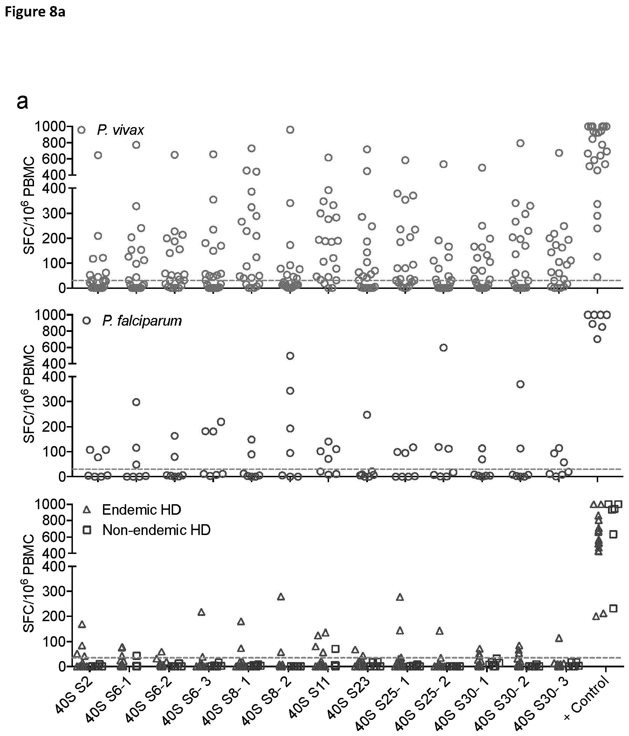

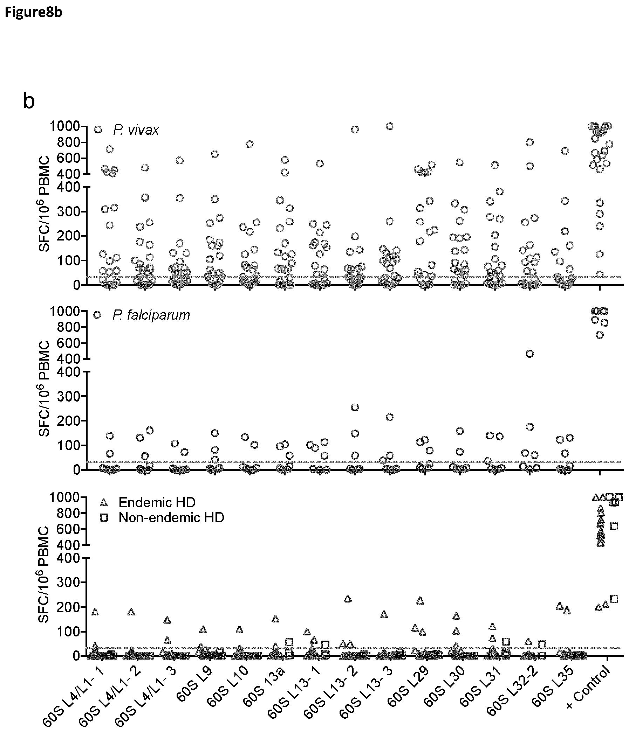

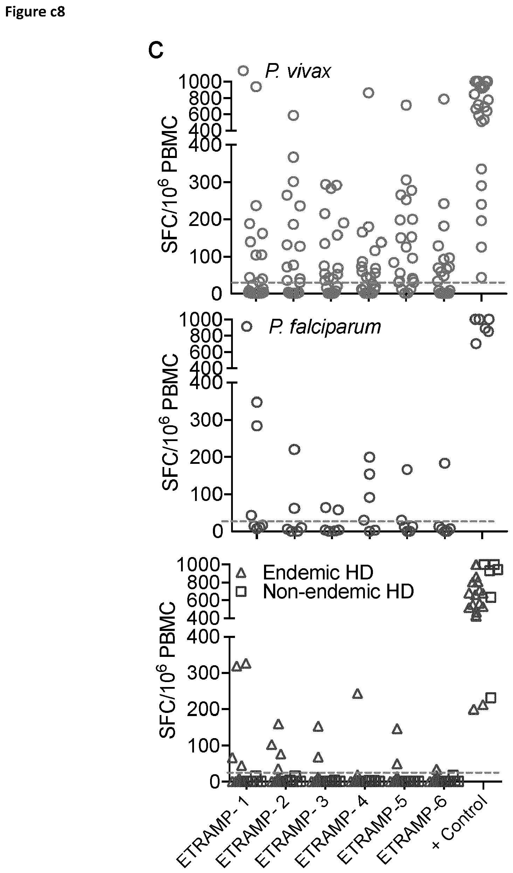

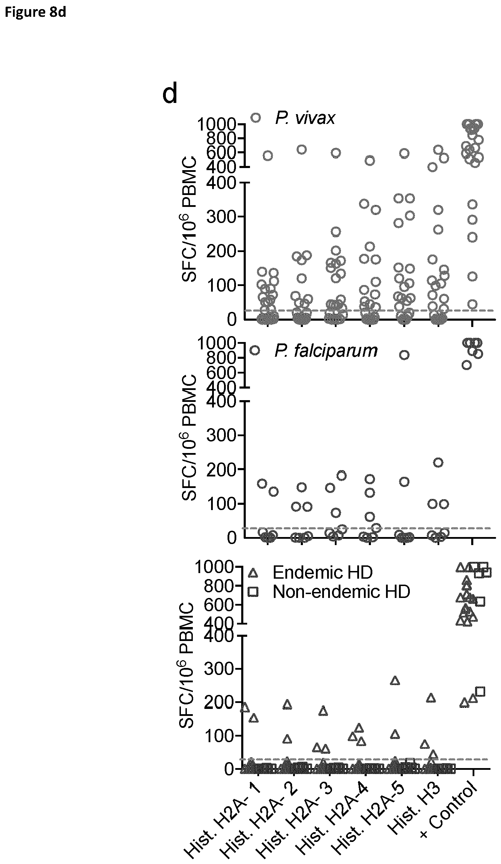

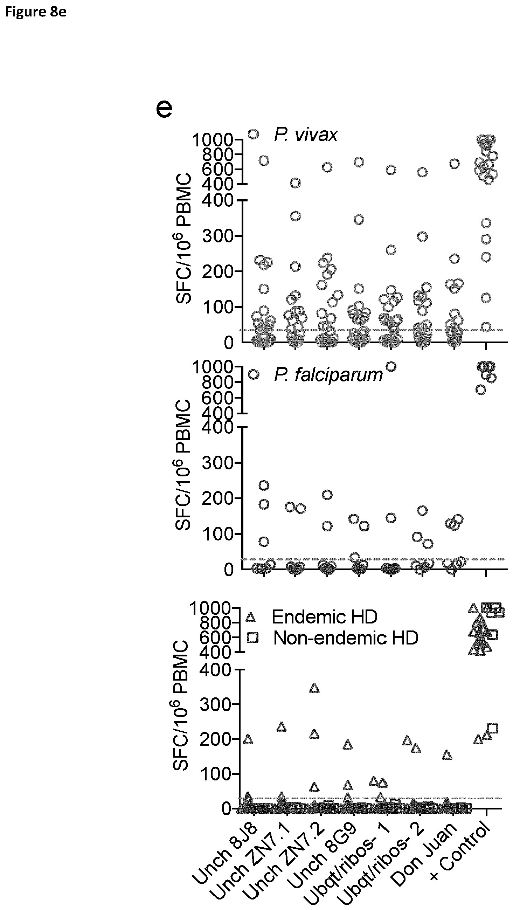

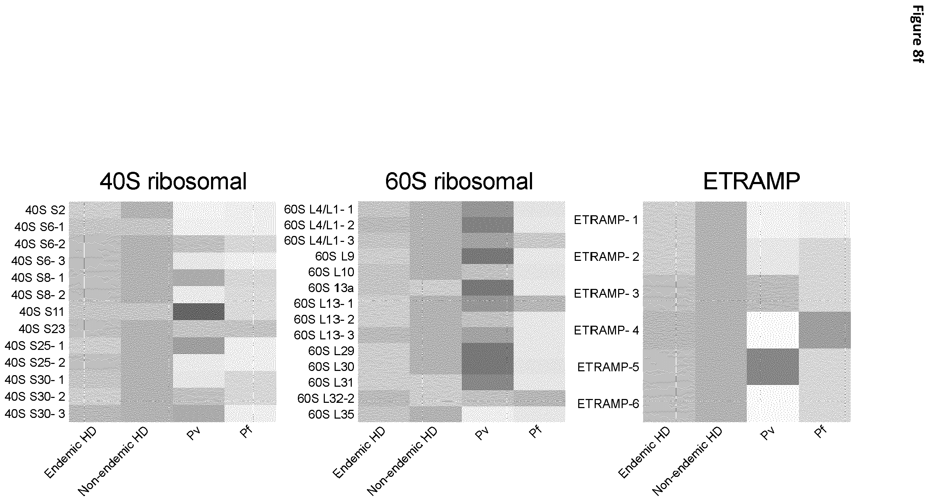

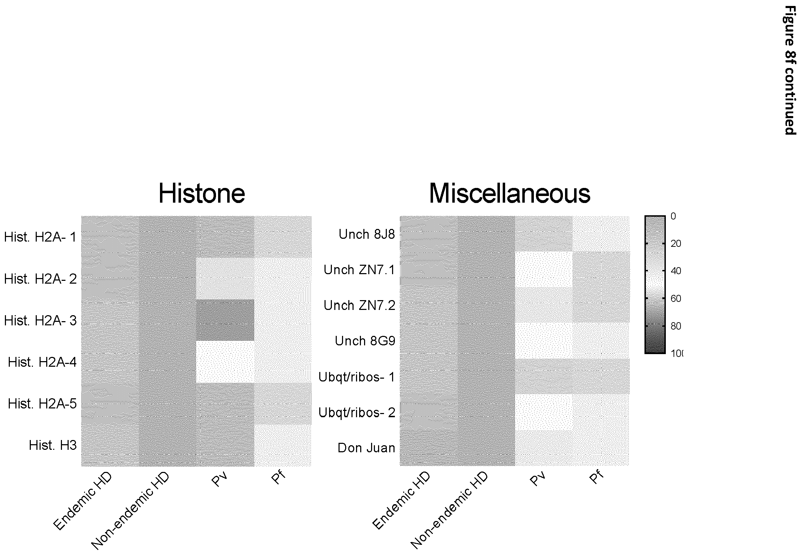

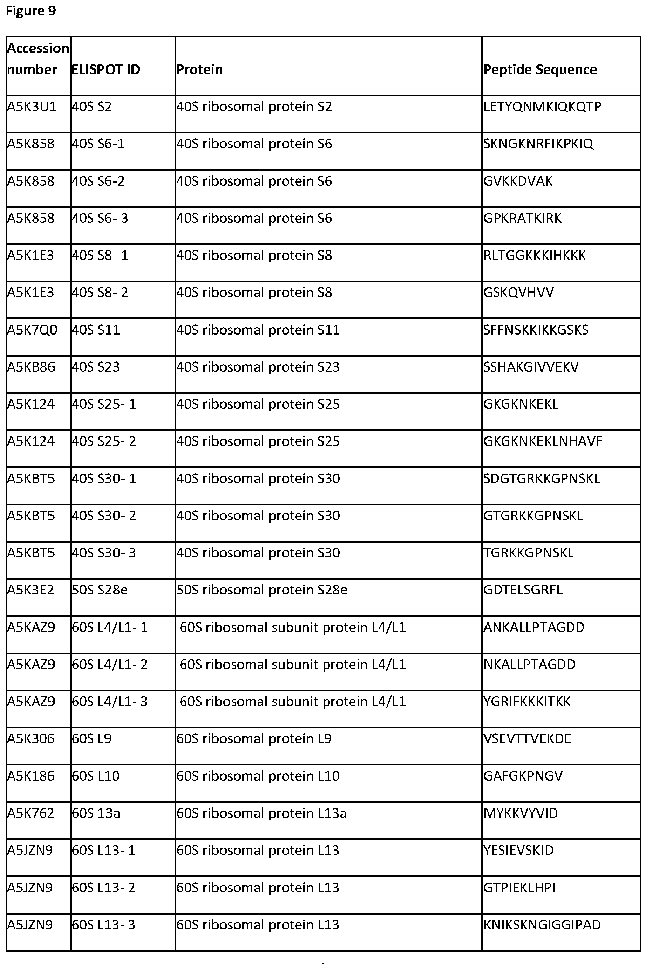

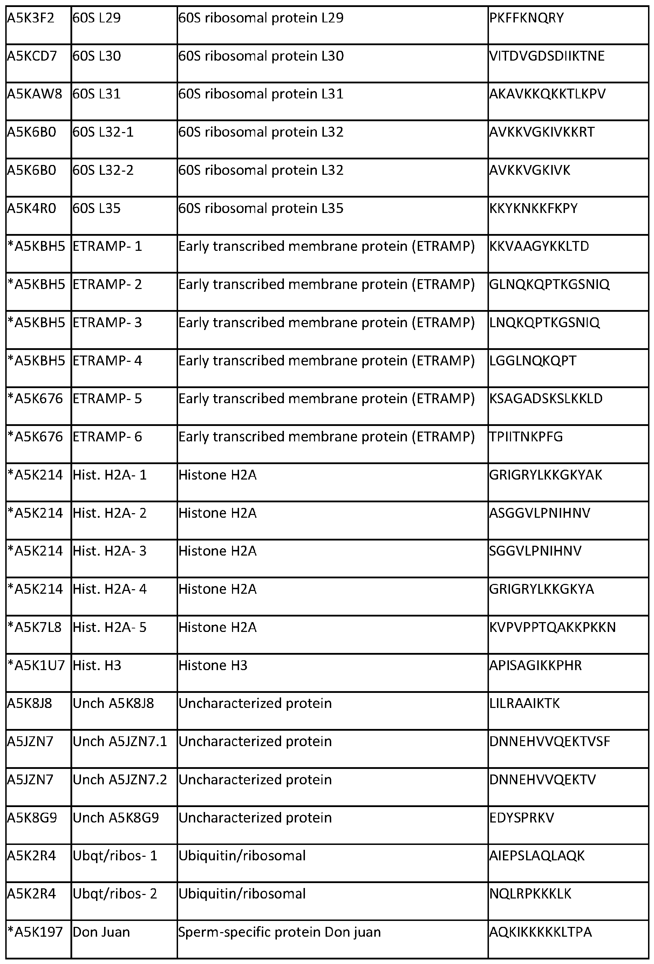

**Figure 8** - demonstrates peptide validation by *ex vivo* ELIspot assay. Selected peptides (see Figure 9) were tested using PBMC isolated from patients infected with *P. vivax* (n = 24), *P. falciparum* (n=7) and healthy donors from endemic (n = 15) and non-endemic (n = 6) regions for malaria. Cells were stimulated with 40S ribosomal peptides (a), 60S ribosomal peptides (b), ETRAMP peptides (c), histone peptides (d) and other peptides (e). Each symbol represents one individual. The circles in the top graphs in Figures 7a, b, c, d and e represent *P*. *vivax* patients, the circles in the middle graphs in Figures 7a, b, c, d and e represent *P. falciparum* patients, the triangles in the bottom graphs in Figures 7a, b, c, d and e represent endemic healthy donors and the squares in the bottom graph in Figures 7a, b, c, d and e represent non-endemic healthy donors. IFN-y production was measured by spot counting and the results are expressed as spot-forming cells (SFC) by 1×10⁶ PBMCs. Positive peptides were considered as responses that induced ≥30 spots in each patient - above the dashed horizontal line. Percentage of positive individuals for each tested peptide in each infected or healthy group are depicted by colour intensity with the legend indicated at the right of the figure (f). Red, yellow, and green represent the high, middle, or poor levels of responders in the ELIspot assay.
**Figure 9** - this table details the peptides used in the ELIspot assays in Figure 8. Non-ribosomal associated proteins are marked with an asterix (*)

### MATERIALS AND METHODS

### 1. Humanised mice infected with P. falciparum

TK-NOG mice were transplanted/engrafted with human primary hepatocytes, which repopulate the damaged liver (repopulation (60-80%) as described in Soulard, V. et al. Nat Commun 6 (2015). This model allows the complete hepatic development of *P. falciparum* and the transition to erythrocytic stages, including the appearance of mature gametocytes. This mouse model closely mimics the physiological complexity and specificity of an *in vivo* infection in the human environment and is also a source of fresh human hepatocytes. Every mouse received hepatocytes from the same donor. The donor HLA alleles were HLA-A*03:01, HLA-A^{∗}11:01, HLA-B*40:01, HLA-B*50:01, HLA-C^{∗}03:02 and HLA-C*06:02. Thirteen TK-NOG mice were used in three independent experiments and infected with *P. falciparum* sporozoites in the tail vein as described in the Table 1.

Mice were infected sporozoites from *P. falciparum,* NF54 or NF135 strains, in the tail vein, and livers were harvested at discreet time points post-infection as described in Table 1 below.

**Table 1: Experimental details of humanised mice infected with P. falciparum**

| Sample number | Experiment number | *P*. *falciparum* strain | Number of *P. falciparum* sporozoites (10^6) | Liver Harvest (days post-infection) |
|---|---|---|---|---|
| 1 | | NF54 | | Non- |
| | 1 | | None | Infected |
| 2 | | | 3 | 3 |
| 3 | | | 3 | 5 |
| 4 | | NF54 | | Non- |
| | 2 | | None | Infected |
| 5 | | | 3 | 3 |
| 6 | | | 3 | 4 |
| 7 | | | 3 | 5 |
| 8, 9, 10, 11 | | NF135 | | Non- |
| | 3 | | None | Infected |
| 12 | | | 10 | 3 |
| 13 | | | 10 | 5 |

50µm thick liver sections were obtained and histology was performed to determine the rate of infection. The rate of infection was determined by staining the parasites with an anti-HSP70 antibody and by calculating the average of schizonts counted, divided by the total number of human hepatocytes in each liver. The total number of human hepatocytes infected (rate of infection multiplied by the total number of human hepatocytes) was also calculated. The rate of infection in these mice varied from 30,000 to 60,000 per liver.

Starting from 200 mg of liver material, cells were lysed and total proteins were collected for mass spectrometry (MS) analysis. See section 3 for details.

### 2. Human reticulocytes infected with P. vivax

Human reticulocytes from patients infected with *P. vivax* were purified as described in Junqueira, C. et al. Nature medicine 24, 1330-1336 (2018). Seven samples containing *>99% P. vivax*-infected reticulocytes varying from 4.3×10⁷ to 7×10⁸ cells (Table 2), were lysed and total proteins were collected for mass spectrometry analysis. See section 3 for details.

| **Sample ID** | **N° retics** | **Parasitaemia** |
|---|---|---|
| 24 | 5x10⁷ | Pv +++ |
| 26 | 1,1x10⁸ | Pv +++ |
| 28 | 7x10⁸ | Pv +++ |
| 30 | 4,3x10⁷ | Pv ++ |
| 31 | 7,2x10⁷ | Pv ++ |
| 34 | 6,2x10⁷ | Pv +++ |
| 35 | 6,2x10⁷ | Pv ++ |

Parasitemia was determined at the site of sample collection following local clinical protocols.

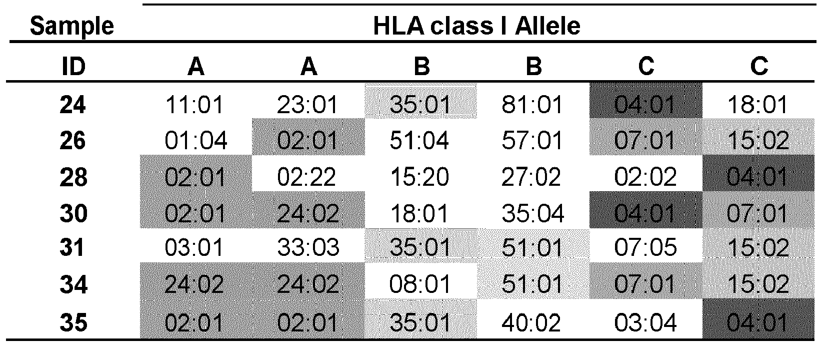

### 3. Peptide identification by immunopeptidomics

### 3.1 Mass spectrometry

Cells were lysed in 1 ml lysis buffer (0.5% Igepal, 150 mM NaCl, 50 mM Tris, pH 8.0, supplemented with complete^{™} protease inhibitor cocktail (Roche)). HLA complexes were immunoprecipitated using 1 mg monoclonal antibody W6/32 against HLA-ABC complexes (GE healthcare) cross-linked to Protein A Sepharose beads using dimethyl pimelimidate (DMP, Sigma). Lysates were incubated overnight. Beads were subsequently washed with 10 column volumes of 2x150 mM NaCl in 50 mM Tris, 1x450 mM NaCl in 50 mM Tris and 50 mM Tris buffer without salt. Peptides bound to the HLA groove are released after mild acid elution with 5 mL 10% acetic acid to denaturate of the alpha-chains and beta-2-microglobulin. The HLA-bound peptides were further purified from beta-2-microglobulin and alpha-chains by HPLC (Ultimate 3000) on a ProSwift RP-1S 4.6 × 50 mm column (Thermo Scientific) by applying a linear gradient of 2-35% (v/v) acetonitrile in 0.1% (v/v) formic acid in water over 10 min. Alternating fractions that did not contain beta-2-microglobulin or alpha-chains were pooled into two final fractions, concentrated and kept at -80°C prior to MS analysis.

Peptides were suspended in 20 µl buffer A (1% acetonitrile, 0.1% TFA in water) and analyzed by nUPLC-MS/MS using an Ultimate 3000 RSLCnano System coupled with an Orbitrap Fusion Lumos Tribrid mass spectrometer (Thermo Scientific). 9 µl of each sample was injected and trapped onto a 3 µm particle size 0.075 mm × 150 mm Acclaim PepMap RSLC column at 8 µl/min flowrate. Peptide separation was performed at 40oC by applying a 1h linear gradient of 3-25% (v/v) acetonitrile in 0.1% (v/v) formic acid, 5% DMSO in water at a flow rate of 250 µl/min on a 2 µm particle size, 75 µm × 50 cm Acclaim PepMap RSLC column. For HLA class II samples, a linear gradient of 5-30% (v/v) acetonitrile was applied. Peptides were introduced to a Fusion Lumos mass spectrometer (Thermo Scientific) via an Easy-Spray source at 2000 V. The ion transfer tube temperature was set to 305oC. Measurement of precursor peptides was performed with a resolution of 120,000 for full MS (300-1500 m/z scan range) at an AGC target of 400,000. Precursor ion selection and fragmentation by high-energy collisional dissociation (HCD at 28% collision energy for charge state 2-4, 35% for charge state 1) was performed in TopSpeed mode at an isolation window of 1.2 Da for singly to quarterly charged ions at a resolution of 30,000 and an AGC target of 300,000 in the Orbitrap for a cycle duration of 2 s. Singly charged ions were acquired with lower priority.

### 3.2 Peptide identification

MS data was analyzed with Peaks 8 (Bioinformatics Solutions and Tran, N. H. et al. Nature methods 16, 63-66 (2019)) for identification of peptide sequences. Spectra were matched to all reviewed human proteins combined with *Plasmodium falciparum* (isolate 3D7) or *Plasmodium vivax* (Salvador I), produced based on UniProt proteomes (UniProt, C. UniProt: a worldwide hub of protein knowledge. Nucleic acids research 47, (2019)). The results were filtered using a score cut-off of -1g10P=15. The searches were performed with the following parameters: no enzyme specificity, no static and variable modifications, peptide tolerance: ± 5ppm and fragment tolerance: ± 0.03 Da.

Human sequences were disregarded from the analysis. The peptide spectrum matches (PSMs) of plasmodial origin obtained were analysed following a pipeline consisting of: 1) Size exclusion: MHC-I peptides longer than 15 amino were excluded. Peptides smaller than 8 amino acid long were excluded; 2) A Peaks score cut-off of 15 was applied to all samples; 3) False positive peptides, that is, peptides with incorrect identification were removed from the samples; 4) A stringent blast analysis was performed in every peptides sequence using the DeBosT script (see section 3.3). Peptides with two or more amino acid different from human sequences were considered non-human and therefore identified as plasmodial peptides. Peptides with higher netMHC rank were prioritized. The amino acids leucine (L) and isoleucine (I) are isomers, which are indistinguishable from each other through the mass spectrometry protocol. All peptides were blasted for all possible combinations of I and L, and when a combination matched a human peptide, the sequence was excluded. Applying these four-step data analysis criteria, a list of plasmodium peptides were identified, see Figure 2 and Figure 3.

### 3.3 DeBosT script

Blast searches (National Library of Medicine, https://blast.ncbi.nlm.nih.gov) of putative plasmodial sequences were performed using a batch script. Sequences that have less than two amino acid differences compared to human sequences were excluded from downstream analysis (Bettencourt, P. et al. Identification of antigens presented by MHC for vaccines against tuberculosis. npj Vaccines 5, 2 (2020)).

### 3.4 Peptide validation

**Malaria infected patients and healthy donors.** *P. vivax* infected patients were recruited in the Tropical Medicine Research Center (Porto Velho-Brazil) along with healthy donors from the same endemic region and from a non-endemic region (Belo Horizonte- Brazil). All participants provided written informed consent for participation in the study, which has a protocol approved by the Institutional Review Boards of the Oswaldo Cruz Foundation and National Ethical Council (CAAE: 59902816.7.0000.5091). Samples were collected at three different times. Firstly, 7 samples from *P. vivax* infected patients were obtained for the mass spec experiments. Twenty two samples from *P. vivax* infected patients, eighteen healthy donors from the endemic region and 6 from a non-endemic region posteriorly were collected for the Elispot assay.

**PBMC and *P. vivax* infected reticulocytes obtention.** 100mL of blood was collected from infected individuals and controls. First, mononuclear cells were separated from peripheral blood (PBMCs). For this, the blood was diluted in a 1: 1 ratio, was gently added to a tube containing 15 mL of Ficoll (GE Healthcare, USA). Red blood cell pellet resulting from PBMC purification was resuspended in RPMI culture medium in a 1:4 ratio. Diluted blood was added carefully into a 50mL tube containing Percoll 45% (GE Healthcare, USA), 5x the volume of the red blood cell pellet. Samples were centrifuged for 15 minutes at 2000 rpm. After centrifugation, the reticulocyte interface was collected. Reticulocyte purified samples had 99% purity.

**ELISPOT assay.** The *ex vivo* IFN-γ ELISpot assays were performed using 5×10⁵ fresh PBMCs from *P. vivax* infected patients, endemic and non-endemic healthy donors. Cells were plated in duplicates into 96-well ELISpot plates (Merck Millipore) precoated with 4µg/ml anti-human-IFN-γ (clone 1-D1K; Mabtech). Peptides of Table 3 (Figure 9) were tested with stimulation of 10µg/ml and stimulated for 18-20 hour at 37 °C under 5% CO₂. Anti-CD3/anti-CD28 antibodies were used as positive control and medium alone as negative control (subtracted from all conditions). After cell removal, plates were developed for 2 hours in the same temperature condition of the stimulus in the presence of 0.2 µg/ml IFN- y, 7-B6-Biotin (Mabtech). Spot detection was performed following incubation for 30 min in the dark with BCIP/NBT Alkaline Phosphatase Substrate (Sigma). Spot-forming cells (SFC) were counted using the ImmunoSpot automated Elispot counter.

**Table 3 - peptides used for ELISpot assay.**

| **Accession number** | **ELISPOT ID** | **Protein** | **Peptide Sequence** |
|---|---|---|---|
| A5K3U1 | 40S S2 | 40S ribosomal protein S2 | LETYQNMKIQKQTP |
| A5K858 | 40S S6-1 | 40S ribosomal protein S6 | SKNGKNRFIKPKIQ |
| A5K858 | 40S S6-2 | 40S ribosomal protein S6 | GVKKDVAK |
| A5K858 | 40S S6- 3 | 40S ribosomal protein S6 | GPKRATKIRK |
| A5K1E3 | 40S S8- 1 | 40S ribosomal protein S8 | RLTGGKKKIHKKK |
| A5K1E3 | 40S S8- 2 | 40S ribosomal protein S8 | GSKQVHVV |
| A5K7Q0 | 40S S11 | 40S ribosomal protein 511 | SFFNSKKIKKGSKS |
| A5KB86 | 40S S23 | 40S ribosomal protein 523 | SSHAKGIVVEKV |
| A5K124 | 40S S25- 1 | 40S ribosomal protein 525 | GKGKNKEKL |
| A5K124 | 40S S25- 2 | 40S ribosomal protein 525 | GKGKNKEKLNHAVF |
| A5KBT5 | 40S S30- 1 | 40S ribosomal protein S30 | SDGTGRKKGPNSKL |
| A5KBT5 | 40S S30- 2 | 40S ribosomal protein S30 | GTGRKKGPNSKL |
| A5KBT5 | 40S S30- 3 | 40S ribosomal protein S30 | TGRKKGPNSKL |
| A5K3E2 | 50S S28e | 50S ribosomal protein S28e | GDTELSGRFL |
| A5KAZ9 | 60S L4/L1- 1 | 605 ribosomal subunit protein L4/L1 | ANKALLPTAGDD |
| A5KAZ9 | 60S L4/L1- 2 | 60S ribosomal subunit protein L4/L1 | NKALLPTAGDD |
| A5KAZ9 | 60S L4/L1- 3 | 60S ribosomal subunit protein L4/L1 | YGRIFKKKITKK |
| A5K306 | 60S L9 | 60S ribosomal protein L9 | VSEVTTVEKDE |
| A5K186 | 60S L10 | 60S ribosomal protein L10 | GAFGKPNGV |
| A5K762 | 60S 13a | 60S ribosomal protein L13a | MYKKVYVID |
| A5JZN9 | 60S L13- 1 | 60S ribosomal protein L13 | YESIEVSKID |
| A5JZN9 | 60S L13- 2 | 60S ribosomal protein L13 | GTPIEKLHPI |
| A5JZN9 | 60S L13- 3 | 60S ribosomal protein L13 | KNIKSKNGIGGIPAD |
| A5K3F2 | 60S L29 | 60S ribosomal protein L29 | PKFFKNQRY |
| A5KCD7 | 60S L30 | 60S ribosomal protein L30 | VITDVGDSDIIKTNE |
| A5KAW8 | 60S L31 | 60S ribosomal protein L31 | AKAVKKQKKTLKPV |
| A5K6B0 | 60S L32-1 | 60S ribosomal protein L32 | AVKKVGKIVKKRT |
| A5K6B0 | 60S L32-2 | 60S ribosomal protein L32 | AVKKVGKIVK |
| A5K4R0 | 60S L35 | 60S ribosomal protein L35 | KKYKNKKFKPY |
| A5KBH5 | ETRAMP- 1 | Early transcribed membrane protein (ETRAMP) | KKVAAGYKKLTD |
| A5KBH5 | ETRAMP- 2 | Early transcribed membrane protein (ETRAMP) | GLNQKQPTKGSNIQ |
| A5KBH5 | ETRAMP- 3 | Early transcribed membrane protein (ETRAMP) | LNQKQPTKGSNIQ |
| A5KBH5 | ETRAMP- 4 | Early transcribed membrane protein (ETRAMP) | LGGLNQKQPT |
| A5K676 | ETRAMP- 5 | Early transcribed membrane protein (ETRAMP) | KSAGADSKSLKKLD |
| A5K676 | ETRAMP- 6 | Early transcribed membrane protein (ETRAMP) | TPIITNKPFG |
| A5K214 | Hist. H2A- 1 | Histone H2A | GRIGRYLKKGKYAK |
| A5K214 | Hist. H2A- 2 | Histone H2A | ASGGVLPNIHNV |
| A5K214 | Hist. H2A- 3 | Histone H2A | SGGVLPNIHNV |
| A5K214 | Hist. H2A- 4 | Histone H2A | GRIGRYLKKGKYA |
| A5K7L8 | Hist. H2A- 5 | Histone H2A | KVPVPPTQAKKPKKN |
| A5K1U7 | Hist. H3 | Histone H3 | APISAGIKKPHR |
| A5K8J8 | Unch A5K8J8 | Uncharacterized protein | LILRAAIKTK |
| A5JZN7 | Unch A5JZN7.1 | Uncharacterized protein | DNNEHVVQEKTVSF |
| A5JZN7 | Unch A5JZN7.2 | Uncharacterized protein | DNNEHVVQEKTV |
| A5K8G9 | Unch A5K8G9 | Uncharacterized protein | EDYSPRKV |
| A5K2R4 | Ubqt/ribos- 1 | Ubiquitin/ribosomal | AIEPSLAQLAQK |
| A5K2R4 | Ubqt/ribos- 2 | Ubiquitin/ribosomal | NQLRPKKKLK |
| A5K197 | Don Juan | Sperm-specific protein Don juan | AQKIKKKKKLTPA |

### 3.5 Spectral match validation

To further confirm the identity of the peptide sequences identified, a spectral match validation experiment will be performed. Synthetic peptides will be produced and compared to a selection of PSMs obtained from the experiments (the biological peptides). Synthetic peptides will be run in the same experimental conditions as biological peptides. The mass over charge [m/z] for each peptide, the charge state, the intensity and distribution of each peak within each peptide sequences, as well as the peptide specific retention time (RT) on the Liquid Chromatography will be compared between synthetic and biological peptides.

### 3.6 CD8+ T-cell response

The CD8+ T-cell responses from healthy patients from endemic and non-endemic areas for malaria, and those of *P. falciparum* and *P. vivax*-infected patients were analysed against peptides identified by immunopeptidomics (Table 3), using IFN-gamma ELISpot. Several peptides, including ribosomal protein peptides were shown to be recognised in *ex vivo* ELISPOT assays. This demonstrated that peptides displayed on MHC-1 molecules on reticulocytes, including those derived from ribosomal proteins, are naturally immunogenic in human infections and thus are good immunogens for vaccination.

### 3.7 Efficacy and immunogenicity of new vaccine candidates

The immunogens described here may be cloned into viral vectors for use as vaccines. The platform of subunit vaccines has proved to be very safe and is very powerful in inducing CD8+ T-cell responses against the antigen that is being expressed. Generation of ChAdOx1, AdHu5 and/or MVA expressing each antigen may be cloned using GeneArt Technology (ThermoFisher Scientific, UK). Subunit vaccines expressing liver stage antigens may be produced. Immunogens may be mammalian codon-optimized flanked by a Kozak consensus sequence, a tPA leader sequence and a GS linker at the 5'-end and at the 3' end, cloned into a GeneArt entry vector and then recombined into an ChAdOx1, AdHu5 and/or MVA destination plasmid as previously described in Dicks, M. D. et al. PloS one 7 (2012) and McConkey, S. J. et al. Nature medicine 9, 729-735 (2003). Efficacy and immunogenicity of the viral vectors will be evaluated in a malaria challenge mouse model.

### EXAMPLES

### Example 1

*Plasmodium vivax* is the most widespread cause of human malaria and the second most lethal after *P. falciparum.* Unusually, *P. vivax* preferentially infects reticulocytes and recently it has been demonstrated that *P*. *vivax*-infected patients have circulating CD8+ T cells that recognize and form immunological synapses with *P. vivax*-infected reticulocytes in an HLA-dependent manner, releasing their cytotoxic granules to kill both host cell and intracellular parasite, preventing reinvasion. 50 and 700 million reticulocytes per subject were obtained from seven Brazilian subjects infected with *P. vivax,* as described in section of the materials and methods.

453 unique peptides were identified by tandem mass spectrometry sequencing and these were from 176 distinct *P. vivax* antigens. There was significant overlap in immunogens identified in the six subjects, with peptides from 29 antigens found in at least 50% of the subjects and peptides from two antigens found in all six subjects, with high quality data. A most striking and unexpected finding was that over half of the peptides (57%) came from a single class of proteins, plasmodial ribosomal or ribosome associated proteins. A list of peptides identified and the protein they are derived from is provided in Figure 2 and Figure 3.

Ribosomal proteins are species-specific and between humans and *Plasmodia,* most ribosomal proteins share approximately 60% sequence identity on average. This divergence provides adequate differences for regions on non-identify between human/mammalian ribosomal and parasite ribosomal sequences to avoid self-tolerance and be suitably immunogenic. However, proteins with less homology to humans are preferred so as to potentially maximise immunogenicity. Furthermore proteins with identify or greater similarity between *P. falciparum* and *P. vivax* are preferred because they are more likely to provide a cross-species protective effect.

Further, ribosomes are required for protein production and their structure and mechanism of polypeptide generation are well understood. Cells and microbes that are rapidly dividing or very metabolically active may need a lot of ribosomes and ribosomal proteins to engage in the required protein synthesis. This is likely to be true of parasitized reticulocytes in which *P. vivax* grows very rapidly. Similarly, within hepatocytes, malaria parasites generally grow very rapidly. For example, in the case of *P. falciparum,* one sporozoite infects a liver cell and seven days later 20,000 parasites with a largely different antigenic composition, malaria merozoites, emerge from the same liver cell. Therefore, the findings of the mass spectrometry analysis support the fact the parasite will need to generate a lot of ribosomal proteins intracellularly, which are capable of ending up on the HLA class I molecules in parasitized cells.

In addition to the vast majority of ribosomal proteins identified in the mass spectrometry analysis of peptides eluted from HLA-I expressed on infected reticulocytes, two Pv ETRAMPs peptides that are expressed both in hepatocyte and reticulocyte stages were also identified. The ETRAMPs compose a family of polymorphic, small, highly-charged transmembrane proteins unique to malaria parasites, they localize in the parasitophorous vacuole membrane (PVM) with the C-terminal region exposed to the RBC cytosol and are also exported to the host cell cytoplasm. Therefore, the ETRAMPs are accessible to the protein machinery that processes and presents endogenous antigens. Furthermore, they are expressed in the first hours of invasion and, thus, the infected reticulocytes may become targets to CTLs at the early stages of infection. In addition, the ETRAMPs are recognized by antibodies from *Plasmodium falciparum* and *Plasmodium vivax* malaria patients and CD4+ T cells from *P. berghei*-infected mice. In conclusion, the HLA-I binding and biology of ETRAMPs suggest that they could be key targets for protective CD8+ T cell-mediated immunity against malaria. Like ribosomal proteins, ETRAMPs have not been employed or evaluated for CD8 + T cell mediated immunity.

### Example 2

As a proof of concept, one plasmodial ribosomal protein gene was selected and expressed to test the concept that plasmodial ribosomal proteins could be suitable immunogens for developing immunogenic, protective malaria vaccines.

The *P. falciparum* 40S ribosomal protein S20e was selected, which is 118 amino acids in length (sequence: MSKLMKGAIDNEKYRLRRIRIALTSKNLRAIEKVCSDIMKG AKEKNLNVS GPVRLPVKTLRITTRK SPCGEGTNTWDRFELRIYKRLIDL YSQCE VVTQMTSINIDPVVEVEVIITDS, Uniprot accession: PF3D7_1003500.1). The protein was expressed in both a simian adenoviral vector ChAdOx1 and in MVA. BALB/c Mice were immunised with a single shot of 1×10^{g} infectious units of the ChAd recombinant and boosted with the MVA three weeks later with a dose of 1×10⁸pfu and challenged intravenously three weeks later with 1000 *P. berghei* sporozoites transgenic for *P. falciparum* 40S ribosomal protein S20e (expressed under a Pbuis4 promoter). There was highly significant protective efficacy with 2 of 8 mice sterilely protected and the remaining six delayed in time to parasitaemia reflecting a substantial reduction of liver parasite load, P = 0. 0001 (Figure 5).

CD-1 mice were immunised with a single shot of 1×10⁸ infectious units of the ChAd recombinant and boosted with the MVA eight weeks later with a dose of 1×10⁸ pfu and challenged intravenously three weeks later with 1000 *P. berghei* sporozoites transgenic for *P. falciparum* 40S ribosomal protein S20e (expressed under a Pbuis4 promoter). There was highly significant protective efficacy with 3 of 10 mice sterilely protected and the remaining six delayed in time to parasitaemia reflecting a substantial reduction of liver parasite load, P = 0. 0001 (Figure 6).

Using prime-target vaccination regime in CD-1 mice, there was highly significant protective efficacy with 5 of 10 mice sterilely protected and the remaining 5 delayed in time to parasitaemia reflecting a substantial reduction of liver parasite load, P = 0. 0001 (Figure 7).

This efficacy with a *Plasmodium falciparum* ribosomal protein immunogen demonstrates that ribosomal protein immunogens are a new class of antigens for malaria vaccination, especially to target the liver-stage of infection.

### Example 3

To allow analysis of larger numbers of *P. falciparum*-infected human hepatocytes, a recently described mouse strain (TK-NOG) in which most of the mouse liver has been replaced by human hepatocytes was utilised (which unlike mouse hepatocytes will support invasion and growth of *P*. *falciparum*)*.*

The TK-NOG mice (Soulard et al Nature Communications 2015) express the HSVtk transgene under the albumin promoter onto the NOD SCID II2Rg/ background. In this mouse strain, the loss of endogenous hepatocytes is inducible by a brief exposure to a non-toxic dose of ganciclovir, a method that is rapid and temporally restricted, and routinely leads to substantial repopulation (60-80%) with human hepatocytes. The human herpes simplex virus thymidine kinase type 1 gene (HSVtk) acts as a conditional lethal marker in mammalian cells. The HSVtk-encoded enzyme is able to phosphorylate certain nucleoside analogs (e.g. ganciclovir, an antiherpetic drug), thus converting them to toxic DNA replication inhibitors. The utility of HSVtk is a conditional negative-selection marker.

These TK-NOG mice were infected with 1×10⁷ *P. falciparum* sporozoites of a rapidly growing strain (e.g. *P. falciparum* NF135) and livers were removed at 3 - 5 days post-infection. After applying the immunopeptidomics pipeline to identify peptides bound to MHC molecules, Table 4 was obtained. Remarkably, the peptide sequence VITDVGDSDIIKTNE that is part of the protein W4IGC0 form *P. falciparum* NF135 (Ribosomal_L7Ae domain-containing protein) (Figure 2 and 3), was also found amongst the peptides eluted from *P. vivax* infected reticulocytes, here designated as protein A5KCD7 (60S ribosomal protein L30) (Table 4). Moreover, another peptide from the protein W4ID94 form *P. falciparum* NF135 (Ribosomal_L23eN domain-containing protein) was identified in this example (Table 4). Peptides from the corresponding homologous protein in *P. vivax* were also identified amongst the peptides eluted from *P*. *vivax* infected reticulocytes, here designated as protein A5K303 (60S ribosomal protein L23a) (Figures 2 and 3).

Finding two antigens in a short list of few confirmed eluted peptides from this type of experiment using human hepatocytes, provides clear evidence that ribosomal protein peptides can be presented on the HLA class I molecules of *P. falciparum*-infected liver cells. This further supports the concept that vaccines based on ribosomal proteins would be effective malaria vaccines.

**Table 4**

| Table 4: Peptides identified by immunopeptidomics in humanized mice infected with *P. falciparum* NF135. | |
|---|---|
| **Peptide** | **Identifier** |
| AAEEGAKAGAL | tr\|W4I970\|W4I970_PLAFA Uncharacterized protein OS=Plasmodium falciparum NF135/5.C10 OX=1036726 |
| | GN=PFNF135 05454PE=4SV=1 |
| DNNNYDDDEII | tr\|W4IEZ4\|W4IEZ4_PLAFA Uncharacterized protein OS=Plasmodium falciparum NF135/5.C10 OX=1036726 |
| | GN=PFNF135 03293 PE=4 SV=1 |
| E(-1801)EYDESGPSIVH R | tr\|W4IC91\|W4IC91_PLAF A Actin-I OS=PlasmodiumfalciparumNF135/5.C10 OX-1036726 GN-PFNF135_04291 |
| | PE=3 SV=1 |
| GGATGAGLAL | tr\|W4IN72\|W4IN72_PLAFA Glycerol-3-phosphate dehydrogenase OS=PlasmodiumfalciparumNF135/5.C10 |
| | OX=1036726 GN=PFNF135 00472 PE=3 SV=1 |
| GK(+42.05)EKTHINLV VIGHVD | tr\|W4IAJ3\|W4IAJ3_PLAFA Elongation factor 1-alpha OS-Plasmodium falciparum NF135/5.C10 OX=1036726 |
| | GN=PFNF135_05027 PE=3 SV=1 |
| HGNNMNTCLM | tr\|W4IGK7\|W4IGK7_PLAFA Uncharacterized protein OS=Plasmodium falciparum NF135/5.C10 OX=1036726 |
| | GN=PFNF135_03672 PE=4 SV=1 |
| KIYEKKILK | tr\|W4IH49\|W4IH49_PLAFA DNA polymerase OS=Plasmodium falciparum NF135/5.C10 OX=1036726 |
| | GN=PFNF135_03009 PE=3 SV=1 |
| KTATLGVI | tr\|W4IGU9\|W4IGU9_PLAFA Uncharacterized protein OS=Plasmodium falciparum NF135/5.C10 OX=1036726 |
| | GN=PFNF135 _ 02418 PE=4 SV=1 :tr\|W4IGJ3\|W4IGJ3_PLAFA Uncharacterized protein OS=Plasmodium falciparum NF135/5.C10 OX=1036726 GN=PFNF135_02842 PE=4 SV=1 |
| LEGKELPG | tr\|W4IHK5\|W4IHK5_PLAFAPhosphoglycerate kinase OS=PlasmodiumfalciparumNF135/5.C10 OX-1036726 |
| | GN=PFNF135 02647PE=3 SV=1 |
| LVITDVGDSDIIKTNE | tr\|W4IGC0\|W4IGC0_PLAFA Uncharacterized protein OS=Plasmodium falciparum NF135/5.C10 OX-1036726 |
| | GN=PFNF135 03035 PE=4 SV=1 |
| NEAEEFEDY | tr\|W4IKH7\|W4IKH7 _PLAFA Uncharacterized protein OS=Plasmodium falciparum NF135/5.C10 OX=1036726 |
| | GN=PFNF135 01561 PE=4 SV=1 |
| PEEVAEELV | tr\|W4IFR1\|W4IFR1_PLAFA Uncharacterized protein OS=Plasmodium falciparum NF135/5 .C10 OX=1036726 |
| | GN=PFNF135 03212 PE=4 SV=1 |
| PQNINEYF | tr\|W4ICP6\|W4ICP6_PLAFA Uncharacterized protein OS-Plasmodium falciparum NF135/5.C10 OX=1036726 |
| | GN=PFNF135 05101 PE=4 SV=1 |
| QGGGSPLLGT | tr\|W4IM59\|W4IM59_PLAF A Uncharacterized protein OS=Plasmodium falciparum NF135/5.C10OX=1036726 |
| | GN=PFNF135 00968 PE=4 S V= 1 |
| QGISDDSSIHH | tr\|W4IBP5\|W4IBP5 _PLAFA Uncharacterized protein OS=PlasmodiumfalciparumNF135/5.C10 OX-1036726 |
| | GN=PFNF135 05175 PE=4 SV=1 |
| SLGSSILTK | tr\|W4IB92IW4IB92 _PLAFA Uncharacterized protein OS=Plasmodium falciparum NF135/5.C10 OX=1036726 |
| | GN=PFNF135 05237 PE=4 SV=1 |
| SLNDALIVSI | tr\|W4IAD7\|W4IAD7_PLAFA Uncharacterized protein O S=Plasmodium falciparum NF135/5 .C10 OX=1036726 |
| | GN=PFNF135 06050 PE=4 SV=1 |
| VANKIGIL | tr\|W4ID94\|W4ID94_PLAFA Uncharacterized protein OS=Plasmodium falciparum NF135/5.C10 OX=1036726 |
| | GN=PFNF135 04649 PE=3 SV=1 |
| VITDVGDSDIIKTNE | tr\|W4IGC0\|W4IGC0_PLAFA Uncharacterized protein OS=Plasmodium falciparum NF135/5C10OX=1036726 |
| | GN=PFNF135 03035 PE=4 SV= 1 |
| VLPELNGK | tr\|W4I9B4\|W4I9B4_PLAFA Glyceraldehyde-3-phosphate dehydrogenase OS=Plasmodium falciparumNF135/5.C10 |
| | OX-1036726 GN-PFNF135 05644 PE=3 SV=1 |

### Example 4

### Peptide validation by ELIspot assay

48 peptides were tested by *ex vivo* IFN-y ELIspot assay. The peptides are detailed in Figure 9. All peptides were tested in *P. vivax, P. falciparum* and Endemic and Non-endemic healthy donors. The peptides were divided into ribosomal peptides, ETRAMP peptides, histone peptides and other peptides.

All ribosomal peptides were immunogenic in the *P. vivax* tested samples and twelve were positive in at least 70% of the patients (Fig. 8a). Peptide 40S S11 for example, was immunogenic in 86% of *P. vivax* samples, and 60S L4/L1-2, 60S L9, 60S L13-a, 60S L13-3 and 60S L29 were positive in 72%. *P. falciparum* samples were positive for nine ribosomal peptides. Among these peptides, three were immunogenic in more than half of *P. falciparum* samples, the 40S S8, 40S S11, 60S L13-1 and the 60S L32-2. Only one healthy donor individual from a non-endemic area showed a positive response to seven ribosomal peptides, whereas in healthy donors from an endemic malaria region, only 26% of the individuals showed a positive response after stimulation with ribosomal peptides.

All the ETRAMP peptides tested were immunogenic in the *P. vivax* samples, ranging from 47.8 to 82.6% positivity in the tested patients (Fig. 8c). Regarding *P. falciparum,* from six ETRAMP peptides tested, three showed a positive response for at least half of the patients. The higher positivity in healthy donors from an endemic area was 26%, while healthy donors from a non-endemic area did not show a positive response to any peptide.

All the histone peptides tested were immunogenic in all *P. vivax* and all *P. falciparum* tested samples. In *P. vivax* the rate of responder patients ranged between 40% and 71.4% (Fig. 8d) and P. *falciparum* at least two in seven patients obtained a positive response to these peptides. No healthy individual from a non-endemic area showed a positive response, whereas the rate of positivity in healthy individuals from an endemic area was only 13.33%.

The Uncharacterized proteins or Don Juan peptides showed a rate of patients with positive responses between 45 and 63.6% (Figure 8e). From the groups of patients infected with *P. falciparum* or from healthy donors in an endemic malaria region, a positive response was observed in one individual for all the peptides tested. No individual from a non-endemic area showed a positive response to these peptides.

## Claims

1. An immunogenic composition comprising:
a) one or more plasmodium-derived ribosomal or ribosomal associated protein, peptide or immunogenic fragment thereof which has:
i) a sequence which is at least about 80%, 85%, 90%, 95%, 98%, 99% or 100% identical to a 40S or a 60S ribosomal protein, or an immunogenic fragment of a 40S or a 60S ribosomal protein, recited in Figure 1; or
ii) a sequence which is at least about 80%, 85%, 90%, 95%, 98%, 99% or 100% identical to a 40S or a 60S ribosomal protein or peptide thereof, or immunogenic fragment of a 40S or a 60S ribosomal protein or peptide thereof, recited in Figure 2 or Figure 3;
wherein the 40S ribosomal protein is other than the 40S ribosomal protein S20e; and/or
b) a polynucleotide encoding one or more protein, peptide or immunogenic fragment of a);
wherein the immunogenic composition is for use in eliciting an immune response in a subject to treat or prevent malaria.

2. The immunogenic composition of claim 1, wherein the one or more plasmodium-derived ribosomal or ribosomal associated protein, peptide or immunogenic fragment thereof is derived from *Plasmodium falciparum* and/or from *Plasmodium vivax.*

3. The immunogenic composition of claim 1 or claim 2, wherein the immune response elicited is a protective immune response, preferably a CD8+ T-cell response.

4. The immunogenic composition of any of claims 1 to 3, wherein the plasmodium-derived ribosomal or ribosomal associated protein, peptide or immunogenic fragment thereof has a sequence which is at least about 80%, 85%, 90%, 95%, 98%, 99% or 100% identical to a protein or a fragment of a protein selected from the group consisting of:
60S ribosomal subunit protein L4/L1, putative (Accession A5KAZ9);
40S ribosomal protein S25, putative (Accession A5K124);
40S ribosomal protein S2, putative (Accession A5K3U1);
60S ribosomal protein L9, putative (Accession A5K306);
60S ribosomal protein L30, putative (AccessionA5KCD7);
40S ribosomal protein S23, putative (Accession A5KB86);
40S ribosomal protein S30 (Accession A5KBT5);
60S ribosomal protein L29 (Accession A5K3F2);
40S ribosomal protein S6, putative (Accession A5K858);
40S ribosomal protein S8 (Accession A5K1E3);
60S ribosomal protein L13, putative (Accession A5JZN9);
60S ribosomal protein L23a, putative (Accession A5K303);
40S ribosomal protein S11, putative (Accession A5K7Q0);
60S ribosomal protein L10, putative (Accession A5K186);
60S ribosomal protein L35, putative (Accession A5K4R0); and
Ribosomal protein L37 (Accession A5KA70).

5. The immunogenic composition of any of claims 1-4, further comprising a pharmaceutically acceptable excipient or carrier.

6. The immunogenic composition of any of claims 1-5, wherein the polynucleotide is provided in a vector.

7. The immunogenic composition of claim 6, wherein the vector is a viral vector, DNA vector or RNA vector, preferably wherein the viral vector comprises a Modified Vaccinia Ankara (MVA) virus or an adenovirus.

8. The immunogenic composition of claim 6, wherein the vector comprises a trypanosomatid vector.

9. The immunogenic composition of any of claims 1 to 8, wherein the composition comprises two or more different vectors and/or two or more ribosomal or ribosomal associated proteins, peptides or immunogenic fragments thereof.

10. An immunogenic composition of any of claims 1 to 9 for use in treating or preventing malaria in a subject.

11. One or more plasmodium-derived protein, peptide or immunogenic fragment thereof which has a sequence which is at least about 80%, 85%, 90%, 95%, 98%, 99% or 100% identical to a 40S or a 60S ribosomal protein or a fragment of a 40S or a 60S ribosomal proteinrecited in Figure 1, or a 40S or a 60S ribosomal protein or a peptide thereof or a fragment of a 40S or a 60S ribosomal protein or peptide thereof recited in Figure 2 or Figure 3,
wherein the 40S ribosomal protein is other than the 40S ribosomal protein S20e,
for use in eliciting an immune response in a subject.

12. The one or more plasmodium-derived protein, peptide or immunogenic fragment thereof for the use of claim 11 wherein immune response in a subject is to treat or prevent malaria.

13. An immunogenic composition comprising:
a) one or more plasmodium-derived protein, peptide or immunogenic fragment thereof which has a sequence which is at least about 80%, 85%, 90%, 95%, 98%, 99% or 100% identical to a 40S or a 60S ribosomal protein or a fragment of a 40S or a 60S ribosomal proteinrecited in Figure 1, or a 40S or a 60S ribosomal protein or a peptide thereof or a fragment of a 40S or a 60S ribosomal protein or a peptide thereof recited in Figure 2 or Figure 3; and/or
b) a polynucleotide encoding one or more plasmodium-derived protein, peptide or immunogenic fragment thereof which has a sequence which is at least about 80%, 85%, 90%, 95%, 98%, 99% or 100% identical to a 40S or a 60S ribosomal protein or a fragment of a 40S or a 60S ribosomal protein recited in Figure 1, or a 40S or a 60S ribosomal protein or peptide thereof, or a fragment of a 40S or a 60S ribosomal protein or peptide thereof recited in Figure 2 or Figure 3;
wherein the 40S ribosomal protein is other than the 40S ribosomal protein S20e.

14. A vector comprising a polynucleotide encoding one or more plasmodium-derived protein, peptide or immunogenic fragment thereof which has a sequence which is 80%, 85%, 90%, 95%, 98%, 99% or 100% identical to a 40S or a 60S ribosomal protein or a fragment of a 40S or a 60S ribosomal protein recited in Figure 1, or a 40S or a 60S ribosomal protein or a peptide thereof, or a fragment of a 40S or a 60S ribosomal protein or peptide thereof recited in Figure 2 or Figure 3;
wherein the 40S ribosomal protein is other than the 40S ribosomal protein S20e.

15. The vector of claim 14, wherein the vector is a viral or trypanosomatid vector.
